(19) Europäisches Patentamt
European Patent Office
Office européen des brevets

(11) EP 1 417 189 B1

(12) **FASCICULE DE BREVET EUROPEEN**

(45) Date de publication et mention de la délivrance du brevet:
**14.12.2005 Bulletin 2005/50**

(21) Numéro de dépôt: **02794623.5**

(22) Date de dépôt: **05.08.2002**

(51) Int Cl.[7]: **C07D 277/46**, C07D 277/40, C07D 277/42, A61K 31/425, C07D 417/12 // (C07D417/12, 277:00), C07D211:00

(86) Numéro de dépôt international:
**PCT/FR2002/002802**

(87) Numéro de publication internationale:
**WO 2003/014095 (20.02.2003 Gazette 2003/08)**

(54) **DERIVES D'ACYLAMINOTHIAZOLE, LEUR PREPARATION ET LEUR UTILISATION EN THERAPEUTIQUE**

ACYLAMINOTHIAZOLDERIVATE, DEREN HERSTELLUNG UND DEREN THERAPEUTISCHE VERWENDUNG

ACYLAMINOTHIAZOLE DERIVATIVES, THEIR PREPARATION AND THERAPEUTIC USE

(84) Etats contractants désignés:
**AT BE BG CH CY CZ DE DK EE ES FI FR GB GR IE IT LI LU MC NL PT SE SK TR**

(30) Priorité: **06.08.2001 FR 0110504**
**12.06.2002 FR 0207222**

(43) Date de publication de la demande:
**12.05.2004 Bulletin 2004/20**

(73) Titulaire: **Sanofi-Aventis**
**75013 Paris (FR)**

(72) Inventeurs:
- **DE COINTET, Paul**
  **F-31400 Toulouse (FR)**
- **DESPEYROUX, Pierre**
  **F-31860 Labarthe sur Lèze (FR)**
- **FREHEL, Daniel**
  **F-31160 Estadens (FR)**
- **GUENET, Chantal**
  **F-67370 Pfettisheim (FR)**
- **HECKEL, Corinne**
  **F-67350 La Walck (FR)**
- **MAFFRAND, Jean-Pierre**
  **F-31120 Portet sur Garonne (FR)**
- **PRUSS, Rebecca**
  **FR - 13260 Cassis (FR)**

(74) Mandataire: **Ludwig, Jacques et al**
**Sanofi-Aventis**
**174 avenue de France**
**75013 Paris (FR)**

(56) Documents cités:
**WO-A-00/19210** **WO-A-98/22433**
**WO-A-98/22494** **WO-A-98/28268**
**WO-A-98/38177**

- **DATABASE CA [en ligne] CHEMICAL ABSTRACTS SERVICE, COLUMBUS, OHIO, US; KOJIMA, SHINICHI ET AL: "Preparation of amino acid and.alpha.,.beta.-didehydroamino acid derivatives as.beta.-amyloid formation inhibitors" retrieved from STN Database accession no. 132:322142 CA XP002193188 -& WO 00 24392 A (SUMITOMO PHARMACEUTICALS COMPANY, LIMITED, JAPAN) 4 mai 2000 (2000-05-04)**

Remarques:
Le dossier contient des informations techniques présentées postérieurement au dépôt de la demande et ne figurant pas dans le présent fascicule.

EP 1 417 189 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

L'invention a pour objet des dérivés d'acylaminothiazole leur préparation et leur utilisation en thérapeutique.

La présente invention a pour premier objet des composés répondant à la formule générale (I) :

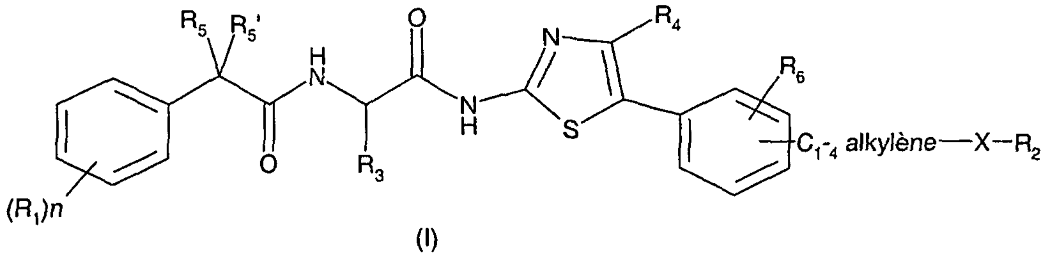

(I)

dans laquelle,

n est égal à 0, 1, 2 ou 3 ;

X représente un atome d'oxygène ou de soufre ;

$R_1$ représente, indépendamment l'un de l'autre lorsque n = 2 ou 3, un atome d'halogène, un hydroxy, un $C_{1-3}$ alkyle, un $C_{1-3}$ alcoxy, un trifluorométhyle, un trifluorométhyloxy ou un méthylènedioxy ;

$R_2$ représente un groupe $C_{1-6}$ alkyle éventuellement substitué par un groupe $C_{3-7}$ cycloalkyle, un phényle, un groupe $C_{1-3}$ alcoxy, un hydroxy ou un atome d'halogène; un groupe $C_{3-7}$ cycloalkyle, pipéridinyle ou phényle ;

les groupes $C_{3-7}$ cycloalkyle, pipéridinyle et phényle étant éventuellement substitués par un ou plusieurs groupes $C_{1-3}$ alkyle, $C_{1-3}$ alcoxy, un hydroxy ou un atome d'halogène ;

$R_3$ représente un atome d'hydrogène ou un groupe $C_{1-6}$ alkyle éventuellement substitué par un groupe $C_{3-7}$ cycloalkyle ;

$R_4$ représente un atome d'hydrogène ou un groupe $C_{1-4}$ alkyle ;

$R_5$ et $R_{5'}$ représentent, indépendamment l'un de l'autre, un atome d' hydrogène, un hydroxy, un atome d'halogène, un groupe $C_{1-3}$ alkyle; ou $R_5$ et $R_{5'}$ forment ensemble un groupe oxo ; et

$R_6$ représente un atome d'hydrogène, un atome d'halogène, un $C_{1-3}$alkyle, un $C_{1-3}$alcoxy, un trifluorométhyle, ou un trifluorométhoxy.

L'art antérieur ne divulgue pas de composés comportant un groupe thiazole substitué par un groupe phényl-$C_{1-4}$-alkylene-X-$R_2$ comme détaillé ci-après.

**WO98128268 (D1)** décrit des composés de formule :

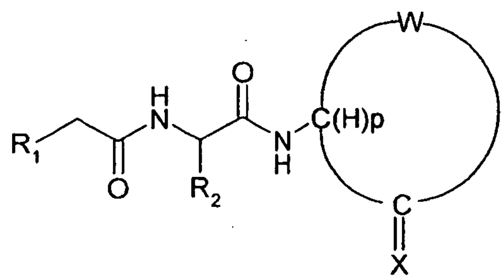

dans laquelle

$R_1$ est un groupe aryle, et plus particulièrement un phényle, substitué ou non substitué;

$R_2$ est un groupe alkyle; et

W avec -C(H)pC(=X) est un cycloalkyle, un cycloalcènyle ou un groupe hétérocyclique substitué ou non substitué, et plus particulièrement un groupe cycloalkyle, un groupe cyclique tel qu'un groupe lactone, lactame, benzazépinone, dibenzazépinone or benzodiazépine (p. 87 l. 1 à 3 de D1).

**WO98/22433 (D2)** décrit des composés de formule:

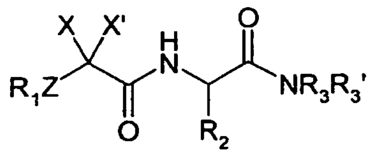

dans laquelle
Z est une liaison ;
X et X' sont des atomes d'hydrogène;
$R_1$ est un groupe aryle, et plus particulièrement un phényle, substitué ou non substitué;
$R_2$ est un alkyle comportant de 1 à 4 atomes de carbone ; et
$R_3$ et $R_3$' sont, indépendamment l'un de l'autre, un atome d'hydrogène, un groupe $-(R_7)n(W)p$ où n peut être égal à 0, p peut être égal à 1 et W est un groupe hétérocyclique.

Dans D2, $R_3$ ou $R_3$' peuvent représenter un groupe thiazole (p. 20, I. 20) qui peut être substitué par un groupe aryle (p. 19, I.21), tel qu'un groupe phényle (p. 18, I. 8).

**WO00124392 (D3)** décrit des composés de formule:

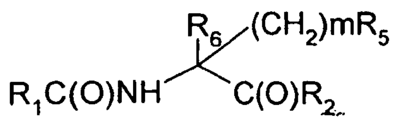

dans laquelle
$R_1$ est un aryle, un hétérocycle ou un alkyle, ces groupes étant éventuellement substitués ;
$R_2$ est un amino, un alcoxy ou un hydroxy, ces groupes étant éventuellement substitués ;
$R_5$ est un aryle ou un hétérocycle, ces groupes étant éventuellement substitués ;
m ést égal à 0,1 ou 2 ; et
$R_6$ est un atome d'hydrogène ou un alkyle.
$R_2$ peut être un groupe $N(R_7)(R_8)$ où l'un des groupes $R_7$ ou $R_8$ est un atome d'hydrogène ou un alkyl et l'autre groupe est un hétérocycle éventuellement substitué tel qu'un 2-thiazolyl.

Parmi les composés de formule générale (I), les composés préférés sont ceux pour lesquels :

- X représente un atome d'oxygène ou de soufre; et/ou
- $R_1$ représente, indépendamment l'un de l'autre lorsque n = 2 ou 3, un atome d'halogène, un méthylènedioxy, un groupe $C_{1-3}$ alkyle, $C_{1-3}$ alcoxy ou un trifluorométhyloxy; plus particulièrement un atome d'halogène ; et/ou
- $R_2$ représente un groupe $C_{1-4}$ alkyle éventuellement substitué par un groupe $C_{4-7}$ cycloalkyle ou phényle ; un groupe $C_{4-7}$ cycloalkyle, pipéridinyle ou phényle ;
  les groupes $C_{4-7}$ cycloalkyle, pipéridinyle et phényle étant éventuellement substitués par un ou plusieurs groupes, de préférence par 1 ou 2 groupes, $C_{1-3}$ alkyle ou $C_{1-3}$ alcoxy ; et/ou
- $R_3$ représente un atome d'hydrogène ou un groupe $C_{1-4}$ alkyle, plus particulièrement un méthyle, éthyle, propyle, éventuellement substitué par un groupe $C_{4-7}$ cycloalkyle; et/ou
- $R_4$ représente un atome d'hydrogène ou un groupe $C_{1-3}$ alkyle, plus particulièrement le méthyle ; et/ou
- $R_5$ et $R_{5'}$ représentent, indépendamment l'un de l'autre, un atome d'hydrogène, d'halogène et plus particulièrement un atome de fluor, un hydroxy ou un groupe $C_{1-3}$ alkyle et plus particulièrement un méthyle ; ou $R_5$ et $R_{5'}$ forment ensemble un groupe oxo ; et/ou
- $R_6$ est un atome d'hydrogène ou un $C_{1-3}$alcoxy et plus particulièrement un méthoxy.

Les composés pour lesquels à la fois X, $R_1$, $R_2$, $R_3$, $R_4$, $R_5$, $R_{5'}$ et $R_6$ sont tels que définis ci-dessus dans les sous-groupes de composés préférés, sont particulièrement préférés et plus spécifiquement parmi ceux-ci les composés pour lesquels:

X représente un atome d'oxygène ; et/ou
$R_1$ représente un atome de fluor en position 3 et un autre atome de fluor en position 5 (n=2).

A titre d'exemple de composés préférés, on peut citer les composés suivants :

**1:** (2S)-2-{[2-(3,5-difluorophényl)acétyl]amino}-*N*-{5-[2-(isopropoxyméthyl)phényl]-1,3-thiazol-2-yl}propanamide
**2:** *N*-(5-{2-[(cyclohexyloxy)méthyl]phényl}-1,3-thiazol-2-yl)-2-{[2-(3,5-difluorophényl)acétyl]amino}propanamide
**3:** 2-{[2-(3,5-difluorophényl)acétyl]amino}-*N*-{5-[2-(éthoxyméthyl)phényl]-1,3-thiazol-2-yl}propanamide
**4:** 2-{[2-(3,5-difluorophényl)acétyl]amino}-*N*-{5-[2-(isobutoxyméthyl)phényl]-1,3-thiazol-2-yl}propanamide
**5:** 2-{[2-(3,5-difluorophényl)acétyl]amino}-*N*-{5-[2-(phénoxyméthyl)phényl]-1,3-thiazol-2-yl}propanamide
**6 :** *N*-(5-{2-[(benzyloxy)méthyl]phényl}-1,3-thiazol-2-yl)-2-{[2-(3,5-difluorophényl)acétyl]amino}propanamide
**7 :** *N*-(5-{2-cyclopentyloxy)méthyl]phényl}-1,3-thiazol-2-yl)-2-{[2-(3,5-difluorophényl)acétyl]amino}propanamide
**8 :** N-(5-{2-[(cyclohexylméthoxy)méthyl]phényl}-1,3-thiazol-2-yl)-2-{[2-(3,5-difluorophényl)acétyl]amino}propanamide
**9 :** N-(5-{2-[(cyclohexyloxy)méthyl]phényl}-1,3-thiazol-2-yl)-2-{[2-(3,5-difluorophényl)acétyl]amino}butanamide
**10 :** N-(5-(2-[(cydohexyloxy)méthyl]phényl)-1,3-thiazol-2-yl)-2-([2-(3,5-difluorophényl)acétyl]amino}-3-méthylbutanamide
**11 :** *N*-(5-{2-[(cyclohexyloxy)méthyl]phényl}-1,3-thiazol-2-yl)-2-[(2-phénylacétyl)amino]propanamide
**12 :** *N*-(5-{2-[(cyclohexyloxy)méthyl]phényl}-4-méthyl-1,3-thiazol-2-yl)-2-{(2-(3,5-difluorophényl)acétyl]amino} propanamide
**13 :** 2-{[2-(3,5-difluorophényl)acétyl]amino}-*N*-{5-[2{méthoxyméthyl)phényl]-1,3-thiazol-2-yl}propanamide
**14 :** *N*-(5-{2-[(cyclopentyloxy)méthyl]phényl}-4-méthyl-1,3-thiazol-2-yl)-2-{[2-(3,5-difluorophényl)acétyl]amino} propanamide
**15 :** *N*-{2-[(5-{2-[(cyclohexyloxy)méthyl]phényl}-1,3-thiazol-2-yl)amino]-2-oxoéthyl}-2-(3,5-difluorophényl)acétamide
**16 :** (25)-*N*-(5-(2-[(cyclohexyloxy)méthyl]phényl)-1,3-thiazol-2-yl)-2-([2-(3-méthylphényl)acétyl]amino}propanamide
**17 :** (25)-*N*-(5-{2-[(cyclohexyloxy)méthyl]phényl)-1,3-thiazol-2-yl)-2-{[2-(4-méthylphényl)acétyl]amino}propanamide
**18 :** (2S)-2-{[2-(3-chlorophényl)acétyl]amino}-*N*-(5-{2-[(cyclohexyloxy)méthyl]phényl}-1,3-thiazol-2-yl)propanamide
**19 :** (2S)-*N*-(5-{2-[(cyclohexyloxy)méthyl]phényl}-1,3-thiazol-2-yl)-2-{[2-(3-fluorophényl)acétyl]amino}propanamide
**20 :** (2S)-*N*-(5-{2-[(cyclohexyloxy)méthyl]phényl}-1,3-thiazol-2-yl)-2-{[2-(4-fluorophényl)acétyl]amino}propanamide
**21 :** (2S)-2-{[2-(3,5-difluorophényl)acétyl]amino}-*N*-{5-[2-(phénoxyméthyl)phényl]-1,3-thiazol-2-yl}propanamide
**22 :** (2S)-2-{[2-(3,5-difluorophényl)acétyl]amino}-*N*-{5-[2-(isopropoxyméthyl)phényl]-1,3-thiazol-2-yl}propanamide
**23 :** (2S)-*N*-(5-{2-[(cyclohexyloxy)méthyl]phényl}-4-méthyl-1,3-thiazol-2-yl)-2-{[2-(3,5-difluorophényl)acétyl]amino}propanamide
**24 :** (2S)-*N*-(5-{3-[(cyclohexyloxy)méthyl]phényl}-1,3-thiazol-2-yl)-2-{[2-(3,5-difluorophényl)acétyl]amino}propanamide
**25 :** (2R)-*N*-(5-{2-[(cyclohexyloxy)méthyl]phényl}-1,3-thiazol-2-yl)-2-{[2-(3,5-difluorophényl)acétyl]amino} propanamide
**26 :** (2S)-*N*-(5-{2-[(cyclohexyloxy)méthyl]phényl}-1,3-thiazol-2-yl)-2-{[2-(3,5-difluorophényl)acétyl]amino} propanamide
**27 :** (2S)-2-{[2-(3,5-difluorophényl)acétyl]amino}-*N*-{5-[2-(isopropoxyméthyl)phényl]-4-méthyl-1,3-thiazol-2-yl} propanamide
**28 :** (2S)-*N*-(5-{2-[(cyclopentyloxy)méthyl]phényl}-4-méthyl-1,3-thiazol-2-yl)-2-{[2-(3,5-difluorophényl)acétyl]amino}propanamide
**29 :** (2S)-2-{[2-(1,3-benzodioxol-5-yl)acétyl]amino}-*N*-(5-{2-[(cyclohexyloxy)méthyl]phényl}-1,3-thiazol-2-yl) propanamide
**30** : (2S)-*N*-(5-{2-[(cyclohexyloxy)méthyl]phényl}-1,3-thiazol-2-yl)-2-{[2-(3,4-difluorophényl)acétyl]amino}propanamide
**31 :** (2S)-*N*-(5-{2-[(cyclohexyloxy)méthyl]phényl}-1,3-thiazol-2-yl)-2-{[2-(3,5-difluorophényl)acétyl]amino}3-méthylbutanamide
**32 :** (2S)-*N*-(5-{2-[(cyclohexyloxy)méthyl]phényl}-1,3-thiazol-2-yl)-2-{[2-(3,5-difluorophényl)acétyl]amino}butanamide
**33 :** (2S)-*N*-(5-{2-[(cyclohexyloxy)méthyl]phényl}-1,3-thiazol-2-yl)-2-{[2-(4-méthoxyphényl)acétyl]amino}propanamide
**34 :** (2S)-*N*-(5-{2-[(cyclohexyloxy)méthyl]phényl}-1,3-thiazol-2-yl)-2-{[2-(3-méthoxyphényl)acétyl]amino}propanamide
**35** : (2S)-*N*-(5-{2-[(cyclohexyloxy)méthyl]phényl)-1,3-thiazol-2-yl)-2-{[2-(3,5-diméthoxyphényl)acétyl]amino} propanamide
**36** : (2S)-*N*-(5-{2-[(cyclohexyloxy)méthyl]phényl}-1,3-thiazol-2-yl)-2-{[2-(3,5-difluorophényl)acétyl]amino}-4-methylpentanamide
**37 :** (25)-*N*-(5-(2-[(cydohexyloxy)méthyl]phényl)-1,3-thiazol-2-yl)-2-{[2-(3,5-difluorophényl)acétyl]amino}-3,3-diméthylbutanamide
**38 :** (2S)-2-{[2-(4-chlorophényl)acétyl]amino}-*N*-(5-{2-[(cyclohexyloxy)méthyl]phényl}-1,3-thiazol-2-ylpropanamide
**39 :** (2S)-*N*-(5-{4-[(cyclohexyloxy)méthyl]phényl}-1,3-thiazol-2-yl)-2-{[2-(3,5-difluorophényl)acétyl]amino}propanamide
**40 :** (2S)-2-{[2-(3,5-difluorophényl)acétyl]amino}-*N*-{5[2-isobutoxyméthyl)phényl]-1,3-thiazol-2-yl}propanamide
**41 :** (2S)-2-{[2-(3,5-difluorophényl)acétyl]amino}-*N*-(5-[2-(éthoxyméthyl)phényl]-1,3-thiazol-2-yl}propanamide
**42 :** (25)-*N*-(5-{2-[(cydohexyloxy)méthyl]phényl}-1,3-thiazol-2-yl)-2-{[2-(3,5-difluorophényl)acétyl]amino}pentanamide
**43 :** (2S)-*N*-(5-{3-[(cyclohexylméthoxy)méthyl]phényl}-1,3-thiazol-2-yl)-2-{[2-(3,5-difluorophényl)acétyl]amino} propanamide
**44 :** (2S)-3-cyclohexyl-*N*-(5-{2-[(cyclohexyloxy)méthyl]phényl}-1,3-thiazol-2-yl)-2-{[2-(3,5-difluorophényl)acétyl] amino}propanamide
**45 :** (25)-2-{[2-(3,5-difluorophényl)acétyl]amino)-*N*-(5-(3-(isopropoxyméthy)phényl}-1,3-thiazol-2-yl)propanamide
**46 :** (2S)-*N*-(5-{4-[(cyctohexylméthoxy)méthyl]phényl}-1,3-thiazol-2-yl)-2-{[2-(3,5-difluorophényl)acétyl]amino} propanamide
**47 :** (2S)-*N*-(5-{2-[(cyclohexyloxy)méthyl]phényl}-1,3-thiazol-2-yl)-2-{[2-(3,5-difluorophényl)acétyl]amino}hexanamide
**48 :** (2S)-*N*-(5-{2-[(cyclohexyloxy)méthyl]phényl}-1,3-thiazol-2-yl)-2-{[2-(3-trifluorométhoxyphényl)acétyl]amino} propanamide
**49 :** (2S)-*N*-(5-{2-[(cyclopentyloxy)méthyl]phényl}-1,3-thiazol-2-yl)-2-{[2-(3,5-difluorophényl)acétyl]amino}propanamide
**50 :** (2S)-*N*-(5-{2-[(cyclohexyloxy)méthyl]phényl}-1,3-thiazol-2-yl)-2-{[(2R)-2-(3,5-difluorophényl)-2-hydroxyacétyl]amino}propanamide
**51 :** (2S)-*N*-(5-{2-[(cyclohexyloxy)méthyl]phényl}-1,3-thiazol-2-yl)-2-{[(2S)-2-(3,5-difluorophényl)-2-hydroxyacétyl]amino}propanamide
**52 :** (2S)-*N*-(5-{2-[(cyclohexyloxy)méthyl]phényl}-4-méthyl-1,3-thiazol-2-yl)-2-{[2-(3,5-difluorophényl)acétyl]amino}pentanamide
**53 :** (2S)-*N*-(5-{2-[(cyclohexyloxy)méthyl]phényl}-4-méthyl-1,3-thiazol-2-yl)-2-{[(2R)-2-(3,5-difluorophényl)-2-hydroxyacétyl]amino} pentanamide
**54 :** (2S)-*N*-(5-{2-[(cyclohexylsulfanyl)méthyl]phényl}-1,3-thiazol-2-yl)-2-{[2-(3,5-difluorophényl)acétyl]amino} propanamide
**55 :** (2S)-*N*-(5-{2-[(cyclohexyloxy)méthyl]phényl}-1,3-thiazol-2-yl)-2-{[(2S)-2-(3,5-difluorophényl)-2-hydroxyacétyl]amino}pentanamide
**56 :** (2S)-*N*-(5-{2-[(cyclohexyloxy)méthyl]phényl}-1,3-thiazol-2-yl)-2-{[(2R)-2-(3,5-difluorophényl)-2-hydroxyacétyl]amino}pentanamide
**57 :** (2S)-*N*-(5-{2-[(cyclohexyloxy)méthyl]phényl}-4-méthyl-1,3-thiazol-2-yl)-2-{[(2S)-2-(3,5-difluorophényl)-2-hydroxyacétyl]amino}pentanamide
**58 :** (25)-*N*-(5-(2-[(cydohexyloxy)méthyl]phényl)-1,3-thiazol-2-yl)-2-{[2-(3,5-difluorophényl)propanoyl]amino}pentanamide
**59 :** (2S)-*N*-(5-{2-[(cyclohexyloxy)méthyl]phényl}-4-éthyl-1,3-thiazol-2-yl)-2-{[2-(3,5-difluorophényl)acétyl]amino} pentanamide
**60 :** (2S)-*N*-(5-{2-[(cyclohexyloxy)méthyl]phényl}-1,3-thiazol-2-yl)-2-{[2-(3,5-difluorophényl)-2-oxoacétyl]amino} pentanamide
**61 :** (2S)-*N*-(5-{2-[(cyclohexyloxy)méthyl]phényl}-1,3-thiazol-2-yl)-2-{[2-(3,5-difluorophényl)-2-oxoacétyl]amino} propanamide
**62 :** (2S)-*N*-(5-{2-[(cyclohexyloxy)méthyl]phényl}-4-éthyl-1,3-thiazol-2-yl)-2-{[2-(3,5-difluorophényl)acétyl]amino} propanamide
**63 :** (2S)-*N*-(5-{2-[(cyclohexyloxy)méthyl]phényl}-4-isopropyl-1,3-thiazol-2-yl)-2-{[2-(3,5-difluorophényl)acétyl] amino}pentanamide
**64 :** (2S)-*N*-(5-{2-[(cyclohexyloxy)méthyl]phényl}-1,3-thiazol-2-yl)-2-{[2-(3,5-difluorophényl)-2-fluoroacétyl]amino} pentanamide

**65 :** (2S)-*N*-(5-{2-[(cyclohexyloxy)méthyl]phényl}-1,3-thiazol-2-yl)-2-{[(2R)-2-(3,5-difluorophényl)-2-fluoroacétyl]amino}propanamide
**66 :** (2S)-*N*-(5-{2-[(cyclohexyloxy)méthyl]phényl}-1,3-thiazol-2-yl)-2-{[(2S)-2-(3,5-difluorophényl)-2-fluoroacétyl]amino}propanamide
**67 :** (2S)-*N*-(5-{2-[(cyclohexyloxy)méthyl]phényl}-1,3-thiazol-2-yl)-2-{[2-(3,5-difluorophényl)-2-fluoroacétyl]amino}pentanamide
**68 :** (2S)-*N*-(5-{2-[(N-méthylpipéridin-4-yloxy)méthyl]phényl}-4-méthyl-1,3-thiazol-2-yl)-2-{[2-(3,5-difluorophényl)acétyl]amino}pentanamide
**69 :** (2S)-2-{[2-(3,5-difluorophényl)acétyl]amino}-*N*-{5-[2-(isopropoxyméthyl)phényl]-4-méthyl-1,3-thiazol-2-yl}butanamide
**70 :** (2S)-2-{[2-(3,5-difluorophényl)acétyl]amino}-*N*-{5-[2-(isopropoxyméthyl)phényl]-1,3-thiazol-2-yl}butanamide
**71 :** (2S)-2-{[2-(3,5-difluorophényl)acétyl]amino}-*N*-{5-[2-(isopropoxyméthyl)phényl]-4-méthyl-1,3-thiazol-2-yl}pentanamide
**72 :** (2S)-2-{[2-(3,5-difluorophényl)acétyl]amino}-*N*-{5-[2-(isopropoxyméthyl)phényl]-1,3-thiazol-2-yl}pentanamide
**73 :** (2S)-2-{[2-(3,5-difluorophényl)acétyl]amino}-*N*-[5-(2-{[(4-méthoxycyclohexyl)oxy]méthyl}phényl]-1,3-thiazol-2-yl}propanamide
**74 :** (2S)-*N*-(5-{2-(cyclohexyloxy)méthyl]phényl}-1,3-thiazol-2-yl)-2-{[2-(3,5-difluorophényl)-2,2-difluoroacétyl]amino}pentanamide
**75 :** (2S)-2-{[2-(3,5-difluorophényl)acétyl]amino}-*N*-(5-{2-(isopropoxyméthyl)-4-méthoxyphényl}-1,3-thiazol-2-yl)pentanamide

Dans le cadre de l'invention, on entend par :

- $C_{1-3}$ une chaîne carbonée qui peut avoir de 1 à 3 atomes de carbone, $C_{3-6}$ une chaîne carbonée qui peut avoir de 3 à 6 atomes de carbone ;
- alkyle, un groupe aliphatique saturé, linéaire ou ramifié; par exemple, un groupe $C_{1-6}$ alkyle représente une chaîne carbonée de 1 à 6 atomes de carbone, linéaire ou ramifiée, plus particulièrement un méthyle, éthyle, propyle, isopropyle, butyle, isobutyle, secbutyle, tertbutyle, de préférence un méthyle, éthyle, propyle ou isopropyle ;
- alkylène, un groupe alkyle divalent ;
- cycloalkyle, un groupe alkyle cyclique, par exemple, un groupe $C_{3-7}$ cycloalkyle représente une chaîne carbonée cyclique de 3 à 7 atomes de carbone, plus particulièrement un cyclopropyle, cyclobutyle, cyclopentyle, cyclohexyle, cycloheptyle, de préférence un cyclopentyle ou cyclohexyle ;
- alcoxy, un groupe alkyloxy à chaîne aliphatique saturée, linéaire ou ramifiée ;
- atome d'halogène, un fluor, un chlore, un brome ou un iode ;
  et
- « $R_5$ et $R_{5'}$ forment ensemble un groupe oxo », le groupe tel que :

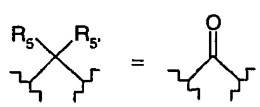

Les composés de formule générale (1) peuvent comporter un ou plusieurs carbones asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.
Les composés de formule (I) peuvent exister à l'état de bases ou de sels d'addition à des acides. De tels sels d'addition font partie de l'invention.
Ces sels sont avantageusement préparés avec des acides pharmaceutiquement acceptables, mais les sels d'autres acides utiles, par exemple, pour la purification ou l'isolement des composés de formule (I) font également partie de l'invention.
Les composés de formule générale (I) peuvent se trouver sous forme d'hydrates ou de solvats, à savoir sous forme d'associations ou de combinaisons avec une ou plusieurs molécules d'eau ou avec un solvant. De tels hydrates et solvats font également partie de l'invention.
Dans ce qui suit, on entend par groupe partant, un groupe pouvant être facilement clivé d'une molécule, avec départ d'une paire électronique, par rupture d'une liaison hétérolytique. Ce groupe peut ainsi être remplacé facilement par un autre groupe lors d'une réaction de substitution par exemple. De tels groupes partants sont, par exemple, les halogènes, ou un groupe hydroxy activé tel qu'un mésylate, tosylate, triflate, acétyle, ...etc. Des exemples de groupes

partants ainsi que des références pour leur préparation sont donnés dans « Advanced Organic Chemistry », J. March, 3rd Edition, Wiley Interscience, p 310-316.

On entend par groupe protecteur, un groupe permettant d'empêcher la réactivité d'une fonction ou position, lors d'une réaction chimique pouvant l'affecter, et qui restitue la molécule après clivage selon des méthodes connues de l'homme du métier. Des exemples de groupes protecteurs ainsi que les méthodes de protection et déprotection sont données, entre autres, dans *Protective groups in Organic Synthesis,* Greene et al., 2nd Ed. (John Wiley & Sons, Inc., New York).

La présente invention a pour second objet des procédés de préparation des composés de formule (I).

Ainsi, ces composés peuvent être préparés par des procédés, illustrés dans les schémas qui suivent, dont les conditions opératoires sont classiques pour l'homme du métier.

Selon le schéma 1, le composé de formule (I) peut être obtenu par couplage peptidique du 2-amino-thiazole de formule (VII) avec l'acylaminoacide de formule (VIII) selon des conditions connues de l'homme du métier, par exemple en présence d'hexafluorophosphate de benzotriazol-1-yloxy-tris(diméthylamino)phophonium (BOP) et de *N*-éthylmorpholine ou *N*-méthyl morpholine dans un solvant inerte tel que le diméthylformamide, l'acétonitrile ou le dichlorométhane à une température pouvant aller de 0°C à la température ambiante.
Le composé de formule (VIII) est obtenu par saponification préalable du composé de formule (IX) selon des méthodes connues de l'homme du métier.

**Schéma 1**

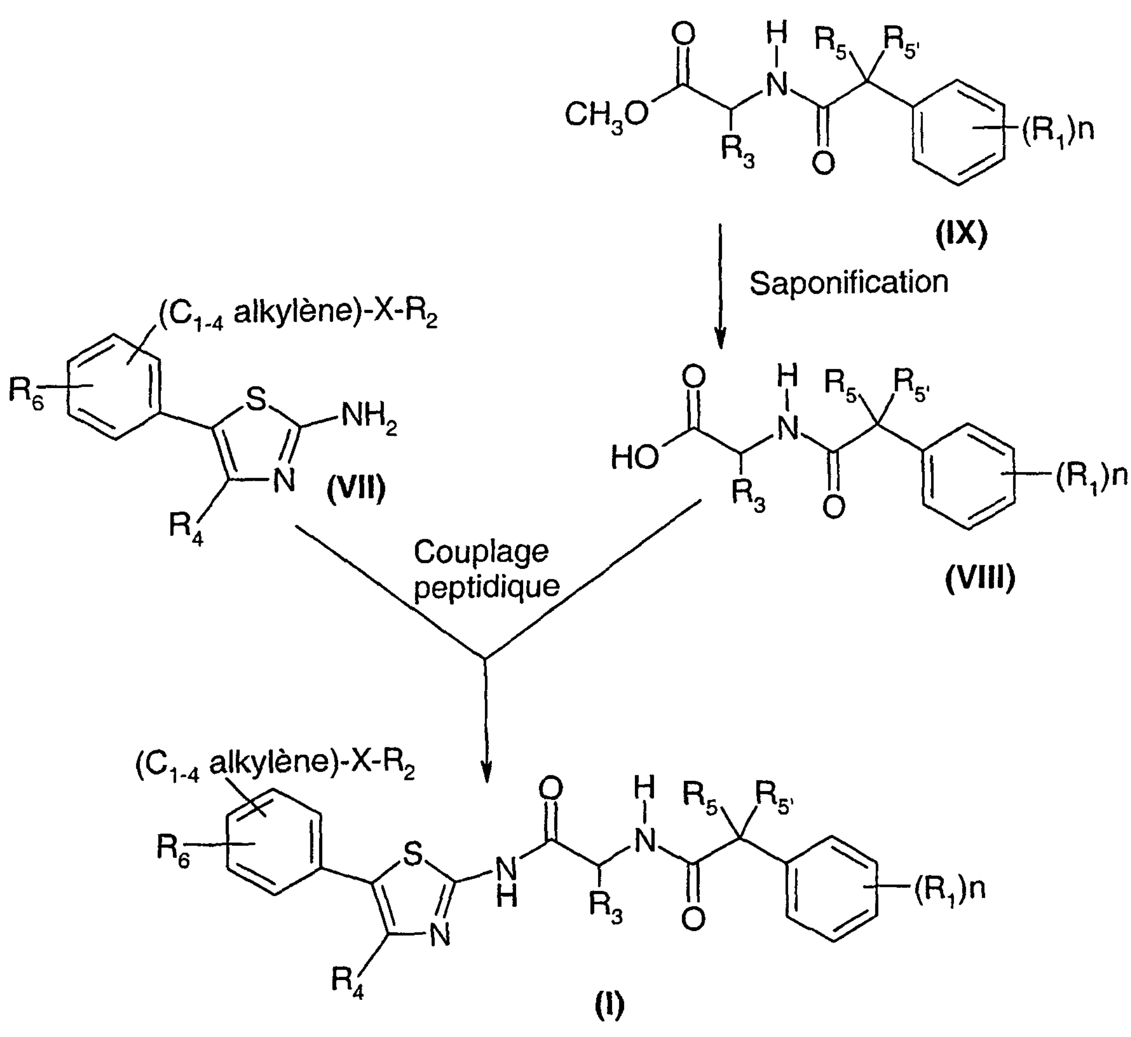


(IX) (VII) (VIII) (I)

Le composé de formule (VII) peut être préparé selon le schéma 2.
Selon ce schéma, les aralkyles de formule (X), dans laquelle Y représente un groupe partant, de préférence un atome d'halogène tel que le brome et Z représente un atome d'halogène tel que le brome, sont condensés avec des alcoolates

ou des thiolates alcalins, par exemple, de formule $R_2X^- Na^{\oplus}$ dans laquelle X représente un atome d'oxygène ou de soufre. La réaction est réalisée dans un solvant inerte tel que le diméthylformamide à une température pouvant aller de 0°C à 50°C, pour conduire aux composés de formule (II). L'aryle de formule (II) est transformé en acide boronique de formule (III) selon une adaptation du procédé décrit par Schoevaars, J. Am. Chem. Soc., 1999, 121, 9550-9561. La transformation peut par exemple être réalisée par formation préalable de l'anion du composé de formule (II), par exemple par action d'une base forte telle que le butyllithium, dans un solvant éthéré tel que le tétrahydrofurane, à des températures pouvant aller de -50°C à -80°C. Cet anion est ensuite mis en réaction avec un borate tel que le triméthylborate pour donner après hydrolyse, l'acide boronique de formule (III).
Le couplage de l'acide boronique (III) avec le thiazole de formule (V) dans laquelle Pg représente un groupe protecteur, tel qu'un imino par exemple plus particulièrement une diphénylcétone imine, peut être effectuée selon la réaction de Suzuki, par une adaptation du procédé décrit par Wolfe, J. Org. Chem., 1997, 62, 4943-4948, pour conduire au 5-phényl-thiazole de formule (VI). Le couplage est réalisé par exemple dans un solvant éthéré tel que le dioxane en présence de phosphate de tripotassium trihydrate et d'un catalyseur tel que le tétrakis(triphénylphosphine) de palladium (0) à une température pouvant aller de la température ambiante à la température de reflux du solvant. Le 5-phényl-thiazole de formule (VI) ainsi préparé est ensuite déprotégé selon des méthodes connues de l'homme du métier pour générer le 5-phényl-2-amino-thiazole de formule (VII).

**Schéma 2**

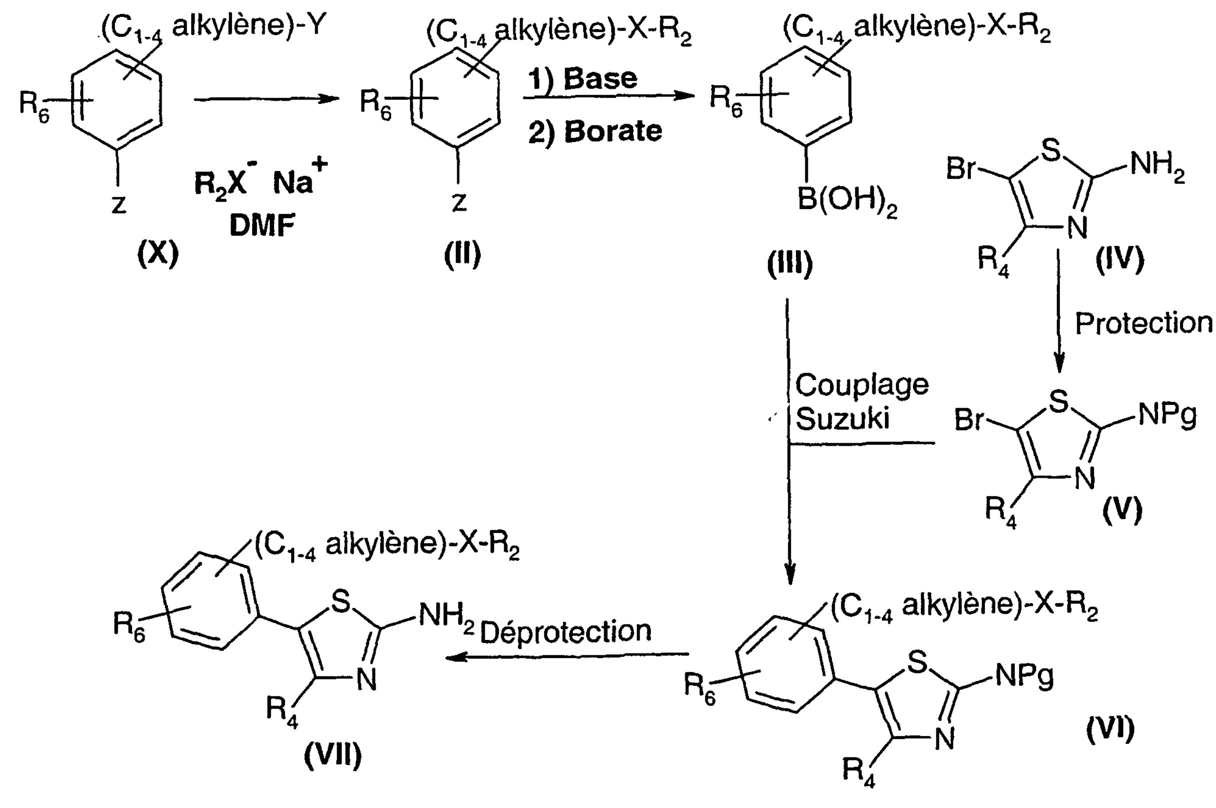


Le 5-bromo-thiazole de formule (V) est obtenu par protection de la fonction amino du composé de formule (IV) correspondant. De préférence, elle est protégée sous forme de diphénylcétone imine dans des conditions connues de l'homme du métier.

Alternativement, les composés optiquement actifs de formule (1), dans laquelle $R_3$ n'est pas un hydrogène, peuvent être obtenus selon le schéma 3 par synthèse stéréospécifique.

Selon ce schéma, le composé de formule (1) peut être obtenu par couplage peptidique de l'amine de formule (XIII) avec l'acide de formule (XIV) selon des conditions connues de l'homme du métier, par exemple en présence d'hexafluorophosphate de benzotriazol-1-yloxy-tris(diméthylamino)-phosphonium (BOP) et de *N*-éthylmorpholine ou *N*-méthylmorpholine dans un solvant inerte tel que le diméthylformamide, l'acétonitrile ou le dichlorométhane à une température pouvant aller de 0°C à la température ambiante.

L'amine de formule (XIII) est obtenue par couplage peptidique de l'amine de formule (VII) avec l'aminoacide de formule (XI), dans laquelle Pg représente un groupe protecteur, dans les conditions telles que décrites ci-dessus,

pour donner le composé de formule (XII). L'aminoacide de formule (XI) est, par exemple, protégé au moyen d'un *N*-tert-butyloxycarbonyl (Boc). Le composé (XII) est ensuite déprotégé selon des méthodes connues de l'homme du métier. Par exemple, si le groupe protecteur utilisé est le Boc, celui-ci peut être déprotégé par hydrolyse acide, en présence d'acide chlorhydrique gazeux anhydre.

Les composés de départ, notamment les composés de formule (IV), (IX), (X), (XI) et (XIV) sont disponibles dans le commerce ou décrits dans la littérature, ou peuvent être préparés par des méthodes qui y sont décrites ou qui sont connues de l'homme du métier.

Par exemple, le 5-bromo-2-amino-thiazole (IV), peut être obtenu par bromation du 2-amino-thiazole correspondant selon une adaptation du procédé décrit par Kaye, J. Chem. Soc. Perkins I, 1981, 2335-2339.

## Schéma 3

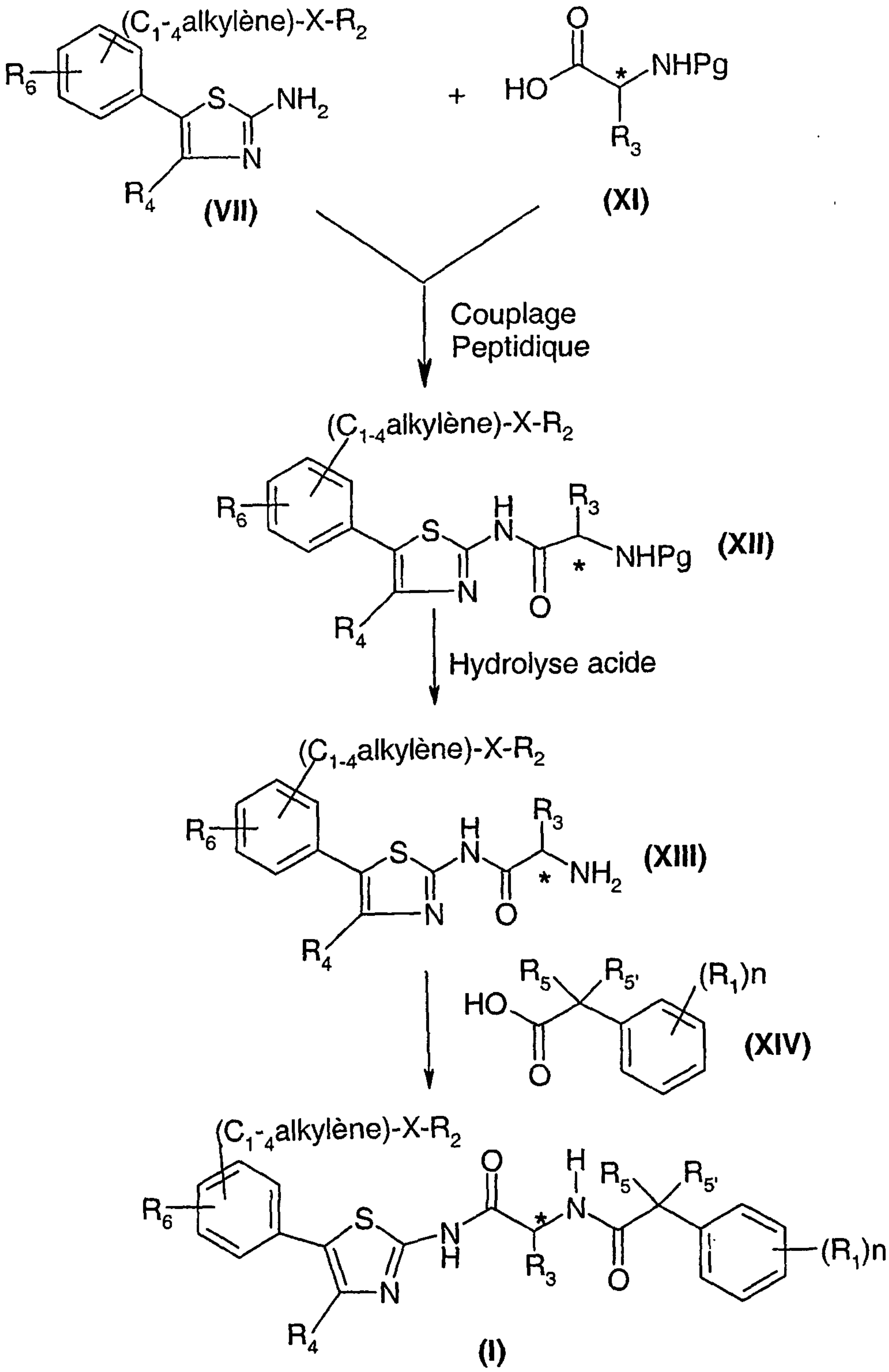


(VII) (XI) (XII) (XIII) (XIV) (I)

* : carbone asymétrique

Par exemple, le composé de formule (IX) peut être obtenu selon les procédés décrits dans les demandes de

brevets WO98/22430 et WO98/22441.
Par exemple, le composé de formule (XIV) peut être obtenu par adaptation des procédés décrits par Middleton et al., J. Org. Chem., 45, 14, 1980, 2883-2887 et par Miyamoto et al., J. Amer. Chem. Soc., 114, 15, 1992, 6256-6257.

Lorsqu'une fonction d'un composé est réactive, par exemple lorsque $R_1$ représente un hydroxy, elle peut nécessiter une protection préalable avant réaction. L'homme du métier pourra déterminer aisément la nécessité d'une protection préalable.

Les significations de n, X, $R_1$, $R_2$, $R_3$, $R_4$, $R_5$, $R_{5'}$ et $R_6$ dans les composés de formule (II) à (XIV) sont telles que définies pour les composés de formule (I).

Les composés de formule (VI) , lorsque -NPg représente une diphénylimine, de formule (VII) et de formule (XIII) sont nouveaux et font également partie de l'invention. Ils sont utiles en tant qu'intermédiaires de synthèse pour la préparation des composés de formule générale (I).

Les exemples suivants décrivent la préparation de certains composés conformes à l'invention.
Les numéros des composés exemplifiés renvoient à ceux donnés dans le tableau ci-après. Les micro-analyses élémentaires et les spectres RMN, IR ou de masse confirment les structures des composés obtenus.

**Exemple 1 :** (2S)-*N*-(5-{2-[(cyclohexyloxy)méthyl]phényl}-1,3-thiazol-2-yl)-2-{[2-(3,5-difluorophényl)acétyl]amino} propanamide (composé n°26).

**Exemple 1.1 :** 1-bromo-2-[(cyclohexyloxy)méthyl]benzène

A 5g de cyclohexanol en solution dans 150ml de diméthylformamide, on additionne à 5°C par portions 1,2g d'hydrure de sodium à 50% en suspension dans l'huile. On agite 1 heure à température ambiante et introduit, à 5°C, 12,5g de bromure de 2-bromobenzyle en solution dans 15ml de diméthylformamide. Après 2 heures à température ambiante, on verse le milieu réactionnel sur de l'eau glacée et on extrait à l'acétate d'éthyle. On sèche la phase organique sur sulfate de sodium anhydre et on l'évapore pour obtenir 13,3g d'huile.
RMN$^{1H}$ $\delta$ en ppm (DMSO d 6) : 1,2-1,95 (massif, 10H) ; 3,45 (m, 1H) ; 4,53 (s, 2H) ; 7,25 (t,1H) ; 7,38 (t, 1H) ; 7,55 (d, 1H) ; 7,62 (d, 1H).

**Exemple 1.2 :** acide 2-[(cyclohexyloxy)méthyl]phénylboronique.

A 13,45g de 1-bromo-2-[(cyclohexyloxy)méthyl] benzène, obtenu à l'étape 1.1, en solution dans 150ml de tétrahydrofurane, on additionne goutte à goutte à
-70°C, 31,2ml de n-butyllithium (1,6 M) en solution dans le n-hexane. Après 2 heures à -70°C, on introduit goutte à goutte 10,2ml de triméthylborate et on laisse remonter la température du milieu réactionnel à -30°C. On hydrolyse avec une solution saturée en chlorure d'ammonium, puis on extrait à l'acétate d'éthyle et on sèche la phase organique avec du sulfate de sodium anhydre. Après évaporation, on obtient 11,7g d'huile jaune.
RMN$^{1H}$ $\delta$ en ppm (DMSO d 6) : 1,15-1,95 (massif, 10H) ; 3,35 (m, 1H) ; 4,61 (s, 2H) ; 7,20-7,35 (massif, 3H) ; 7,52 (d, 1H) ; 8,00 (s, 2H).

**Exemple 1.3 :** 5-bromo-*N*-(diphénylméthylène)-1,3-thiazol-2amine.

A 34g de 5-bromo-1,3-thiazol-2-amine bromhydrate, en suspension dans 300ml de 1,2-dichloroéthane, on additionne 26g de benzophénone imine. On maintient pendant 18 heures à reflux. On filtre le précipité formé et on concentre le filtrat pour obtenir 37,2 g de solide. F=109°C.
RMN$^{1H}$ $\delta$ en ppm (DMSO d 6) : 7,34 (m, 2H) ; 7,50-7,76 (massif, 9H).

**Exemple 1.4**: 5 -{2-[(cyclohexyloxy)méthylphényl}-N-(diphényl méthylène)-1,3-thiazol-2-amine.

A 17g de 5-bromo-*N*-(diphénylméthylène)-1,3-thiazol-2-amine, obtenue à l'étape 1.3, en suspension dans 250ml de 1,4-dioxane, on introduit successivement 24g de phosphate de tripotassium trihydrate, 16,7g d'acide 2-[(cyclohexyloxy)méthyl]phénylboronique, obtenu à l'étape 1.2, 2g de tetrakis(triphénylphosphine) de palladium (0) et on maintient 18 heures à 60°C. On évapore le milieu réactionnel à sec, on le reprend à l'acétate d'éthyle et on le lave à l'eau. On sèche la phase organique sur sulfate de sodium anhydre et on la concentre. On chromatographie le résidu sur une colonne de gel de silice en éluant au dichlorométhane pour obtenir 37,2g d'huile jaune.
RMN $^{1H}$ $\delta$ en ppm (DMSO d 6) : 1,30-1,95 (massif, 10 H) ; 3,40 (m, 1H) ; 4,64 (s, 2H) ; 7,20-7,85 (massif, 15H).

**Exemple1.5** : 5-{-2-[(cyclohexyloxy)méthyl]phényl}-1,3-thiazol-2-amine.

A 11 g de 5-{-2-[(cyclohexyloxy)méthyl]phényl}-*N*-(diphényl méthylène)-1,3-thiazol-2-amine, obtenue à l'étape 1.4, en solution dans 150ml de méthanol, on additionne 75ml d'une solution aqueuse d'acide chlorhydrique (1 M) et on agite 18 heures à 20°C. On évapore à sec, on reprend le résidu à l'éther diéthylique et on lave avec une solution aqueuse d'hydroxyde de sodium (0,5 M). On sèche la phase organique sur sulfate de sodium anhydre et on la concentre. On chromatographie le résidu sur une colonne de gel de silice en éluant avec un mélange dichlorométhane / méthanol 98/2 (v/v), pour obtenir 5,3g d'un solide de couleur beige. F= 102°C.
RMN $^{1H}$ $\delta$ en ppm (DMSO d 6) : 1,15-1,95 (massif, 10H) ; 3,40 (m, 1H) ; 4,51 (s, 2H) ; 7,05 (s, 2H) ; 7,08 (s, 1H) ; 7,25-7,40 (massif, 4H).

**Exemple 1.6 :** *tert*-butyl(1S)-2-[(5-{2-[(cyclohexyloxy)méthyl]phényl}-1,3-thiazol-2-yl)amino]-1-méthyl-2-oxoéthylcarbamate.

A 4g d'acide (2S)-2-[(terbutyloxycarbonyl)amino]propionique en solution dans 35ml de diméthylformamide, on additionne à 0°C, 9g d'hexafluorophosphate de benzotriazol-1-yloxytris(diméthylamino)phosphonium puis, goutte à goutte, 5,5ml de N-éthylmorpholine. Après 15 minutes à cette température, on introduit 3,7g de 5-{-2-[(cyclohexyloxy) méthyl]phényl}-1,3-thiazol-2-amine, obtenue à l'étape 1.5, en solution dans 10 ml de diméthylformamide et on agite 18 heures à température ambiante. On reprend le milieu à l'acétate d'éthyle et on le lave 2 fois à l'eau. On sèche la phase organique sur sulfate de sodium anhydre et on la concentre. On chromatographie le résidu sur une colonne de gel de silice, en éluant avec un mélange dichlorométhane /méthanol 96/4 (v/v) pour obtenir 5,4g d'huile incolore.
RMN $^{1H}$ $\delta$ en ppm (DMSO d 6) : 1,16-1,49 (massif, 18H) ; 1,68-1,88 (massif, 4H) ; 3,36 (m, 1H) ; 4,27 (m, 1H) ; 4,49 (s, 2H) ; 7,25 (d, 1H), 7,37-7,61 (massif, 5H) ; 12,19 (s, 1H).
$[\alpha]_D^{20}$ = -56,7 (c = 1/$CH_3OH$).

**Exemple 1.7 :** chlorhydrate de (2S)-2-amino-*N*-(5-{2-[(cyclohexyloxy) méthyl]phényl}-1,3-thiazol-2-yl)propionamide.

A 4g de *tert*-butyl(1S)-2-[(5-{2-[(cyclohexyloxy)méthyl]phényl}-1,3-thiazol-2-yl)amino]-1-méthyl-2-oxoéthylcarbamate, obtenu à l'étape 1.6, en solution dans 60 ml d'acétate d'éthyle, on additionne goutte à goutte à 0°C, 25 ml d'une solution d'acide chlorhydrique gaz (4,5 N) dans l'éther diéthylique. On agite 18 heures à température ambiante. On filtre le précipité formé, on le rince 2 fois à l'éther diéthylique et on le sèche pour obtenir 2,9g de solide blanc. F= 140°C.
RMN $^{1H}$ $\delta$ en ppm (DMSO d 6) : 1,19-1,48 (massif, 9H) ; 1,66-1,90 (massif, 4H) ; 3,37 (m, 1H) ; 4,18 (m, 1H) ; 4,50 (s, 2H) ; 7,37-7,57 (massif, 4H), 7,65 (s, 1H) ; 8,52 (s, 3H) ; 12,80 (s, 1H).
$[\alpha]_D^{20}$ = + 4,3 (c = 1/$CH_3OH$).

**Exemple 1.8 :** (2S)-*N*-(5-{2-[(cyclohexyloxy)méthyl]phényl}-1,3-thiazol-2yl)-2-{[2-(3,5-difluorophényl)acétyl]amino} propanamide.

A 0,344g d'acide 3,5-difluorophénylacétique en solution dans 10ml de diméthylformamide, on additionne à 0°C, 0,884g d'hexafluorophosphate de benzotriazol-1-yloxytris(diméthylamine)phosphonium, 0,5ml de N-éthylmorpholine et on agite 15 minutes à cette température. On introduit par portion 0,65g de chlorhydrate de (2S)-2-amino-*N*-(5-{2-[(cyclohexyloxy)méthyl]phényl}-1,3-thiazol-2-yl)propionamide, obtenu à l'étape 1.7, et on agite 18 heures à 20°C. On reprend le milieu à l'acétate d'éthyle et on le lave 2 fois à l'eau. On sèche la phase organique sur sulfate de sodium anhydre et on la concentre. On chromatographie le résidu sur une colonne de gel de silice en éluant avec un mélange dichlorométhane / méthanol 98/2 (v/v) pour obtenir après précipitation dans l'eau, 0,73g de solide blanc. F = 140°C.
RMN$^{1H}$ $\delta$ en ppm (DMSO d 6) : 1,15-1,36 (massif, 8H) ; 1,46 (m, 1H) ; 1,64-1,86 (massif, 4H) ; 3,35 (m, 1H) ; 3,56 (s, 2H) ; 4,47 (s, 2H) ; 4,52 (m, 1H) ; 6,98-7,11 (massif, 3H) ; 7,36-7,53 (massif, 4H) ; 7,56 (s, 1H) ; 8,58 (d, 1H) ; 12,27 (s, 1H).
$[\alpha]_D^{20}$ = -138,6 (c = 1/$CH_3OH$).

**Exemple 2 :** (2S)-N-(5-{2-[(cyclohexyloxy)méthyl)phényl}-1,3-thiazol-2-yl)-2-{[(2R)-2-[3,5-difluorophényl)-2-fluoroéthanoyl]amino}propionamide (composé n°65) et (2S)-N-(5-{2-[(cyclohexyloxy)méthyl)phényl}-1,3-thiazol-2-yl)-2-{[ (2S)-2-[3,5-difluorophényl)-2-fluoroéthanoyl]amino}propionamide (composé n°66).

**Exemple 2.1 :** 3,5-difluoromandélate de méthyle

A 2 g d'acide 3,5-difluoromandélique en solution dans 25 ml de méthanol, on additionne 2 ml d'acide sulfurique

à 95% massique et maintient 6 heures à reflux. On concentre le milieu, on reprend le résidu à l'acétate d'éthyle et on le lave avec une solution d'hydroxyde de sodium (0,1 N). On sèche la phase organique sur sulfate de sodium anhydre et on obtient 2 g d'huile.
RMN $^{1H}\delta$ en ppm (DMSO d 6) : 3,70 (s, 3H) ; 5,10 (s, 1H) ; 6,51 (s, 1H) ; 7,11-7,44 (massif, 3H).

**Exemple 2.2 :** (3,5-difluorophényl)fluoroacétate de méthyle

A 2 g de 3,5-difluoromandélate de méthyle, obtenu à l'étape 2.1, en solution dans 25 ml de dichlorométhane, on additionne goutte à goutte à 20°C 2,3g de *N*-éthyl-*N*-(trifluoro-$\lambda^4$-sulfanyl)-1-éthanamine et on agite 18 heures à température ambiante. On reprend le milieu réactionnel au dichlorométhane et on le lave à l'eau, avec une solution aqueuse saturée en hydrogénocarbonate de sodium, puis avec une solution (0,5 N) d'acide chlorhydrique. On sèche la phase organique sur sulfate de sodium anhydre et on la concentre pour obtenir 1,9g d'huile.
RMN$^{1H}$ $\delta$ en ppm (DMSO d 6) : 3,72 (s, 3H) ; 6,28 (d, 1H) ; 7,21-7,42 (massif, 3H).

**Exemple 2.3 :** acide (3,5-difluorophényl)fluoroacétique

A 1,9 g de (3,5-difluorophényl)fluoroacétate de méthyle, obtenu à l'étape 2.2, dans 60ml de méthanol, on additionne 15 ml d'une solution d'hydroxyde de sodium (1N) et on agite 18 heures à température ambiante. On concentre le milieu sous vide, on reprend le résidu à l'acétate d'éthyle et on le lave avec une solution aqueuse (0,5 M) d'acide chlorhydrique. On sèche la phase organique sur sulfate de sodium anhydre et on la concentre pour obtenir 1,6 g d'huile orange.
RMN $^{1H}$ $\delta$ en ppm (DMSO d 6) : 6,10 (d, 1H) ; 7,12-7,39 (massif, 3H).

**Exemple 2.4 :** (2S)-N-(5-{2-[(cyclohexyloxy)méthyl)phényl}-1,3-thiazol-2-yl)-2-{[(2R)-2-(3,5-difluorophényl)-2-fluoroéthanoyl]amino}propionamide et (2S)-N-(5-{2-[(cyclohexyloxy)méthyl)phényl}-1,3-thiazol-2-yl)-2-{[(2S)-2-(3,5-difluorophényl)-2-fluoroéthanoyl]amino}propionamide.

On procède de la même manière que dans l'étape 1.8 de l'exemple 1 en remplaçant l'acide 3,5-difluorophénylacétique par l'acide (3,5-difluorophényl)fluoroacétique, obtenu à l'étape 2.3. On obtient 0,5g du diastéréoisomère (SR) et 0,5g du diastéréoisomère (SS).
**Composé n°65 (SR):**
F = 78°C.
RMN $^{1H}$ $\delta$ en ppm (DMSO d 6) : 1,19-1,88 (massif, 13H) ; 3,38 (m, 1H) ; 4,48 (s, 2H) ; 4,57 (q, 1H) ; 6,07 (d, 1H) ; 7,21-7,59 (massif, 8H) ; 8,96 (d, 1H) ; 12,36 (s, 1H).
$[\alpha]_D^{20}$ = -115,4 (c = 1/$CH_3OH$).
**Composé n°66 (SS) :**
F = 158°C.
RMN $^{1H}$ $\delta$ en ppm (DMSO d 6) : 1,19-1,88 (massif, 13H) ; 3,34 (m, 1H) ; 4,49 (s, 2H) ; 4,57 (q, 1H) ; 6,09 (d, 1H) ; 7,21-7,62 (massif, 8H) ; 8,91 (d, 1H) ; 12,35 (s, 1H).
$[\alpha]_D^{20}$ = -92 (c = 1/$CH_3OH$).

**Exemple 3 :** (2S)-N-(5-{2-[(cycfohexyloxy)méthyl)phényl}-1,3-thiazol-2-yl)-2-{[(2R)-2-[3,5-difluorophényl)-2-hydroxyéthanoyl]amino}propionamide (composé n°50) et (2S)-N-(5-{2-[(cyclohexyloxy)méthyl)phényl}-1,3-thiazol-2-yl)-2-{[(2S)-2-[3,5-difluorophényl)-2-hydroxyéthanoyl]amino}propionamide (composé n°51).

A 0,5 g d'acide 3,5-difluoromandélique en solution dans 25 ml de diméthylformamide, on additionne à 0°C, 1,8g d'hexafluorophosphate de benzotriazol-1-yloxytripyrrolidinophosphonium et 0,45 ml de N-méthylmorpholine. Après 20 min à 0°C, on introduit 0,9g de chlorhydrate de (2S)-2-amino-N-(5-{2-[(cyclohexyloxy)méthyl]phényl}-1,3-thiazol-2-yl)propionamide, obtenu à l'étape 1.7 de l'exemple 1, et on agite pendant 18 heures à 20°C. On verse le milieu réactionnel sur une solution aqueuse d'hydroxyde de sodium (0,5 M) et on extrait à l'acétate d'éthyle. On sèche la phase organique sur sulfate de sodium anhydre et on la concentre. On chromatographie le résidu sur colonne de silice en éluant avec un mélange dichlorométhane/méthanol 99,5/0,5 (v/v) et on obtient 0,22 g de diastéréoisomère (SS) et 0,20g de diastéréoisomère (SR).
**Composé n°50 (SR) :**
F = 169°C.
RMN $^{1H}$ $\delta$ en ppm (DMSO d 6) : 1,18-1,47 (massif, 9H) ; 1,64 (m, 2H) ; 1,86 (m, 2H) ; 3,35 (m, 1H) ; 4,47 (s, 2H) ; 4,53 (m, 1H) ; 5,09 (m, 1H) ; 6,50 (d, 1H) ; 7,12-7,57 (massif, 8H) ; 8,35 (d, 1H) ; 12,28 (s, 1H).
$[\alpha]_D^{20}$ = -106 (c = 1/$CH_3OH$).

**Composé n°51 (SS) :**
F = 159°C.
RMN $^{1H}$ δ en ppm (DMSO d 6) : 1,19-1,48 (massif, 9H) ; 1,65 (m, 2H) ; 1,84 (m, 2H) ; 3,37 (m, 1H) ; 4,48 (s, 2H) ; 4,58 (m, 1H) ; 5,07 (m, 1H) ; 6,54 (d, 1H) ; 7,09-7,60 (massif, 8H) ; 8,59 (d, 1H) ; 12,25 (s, 1H).
$[\alpha]_D^{20}$ = -95 (c = 1/$CH_3OH$).

**Exemple 4 :** (2S)-N-(5-{2-[(cyclohexyloxy)méthyl)phényl}-1,3-thiazol-2-yl)-2-{[2-(3,5-difluorophényl)-2-oxoacétyl]amino}propionamide (composé n°61).

On procède de la même manière que dans l'étape 1.8 de l'exemple 1 en remplaçant l'acide 3,5-difluorophénylacétique par l'acide (3,5-difluorophényl)oxoacétique. On obtient 0,54g du composé. F= 78°C.
RMN $^{1H}$ δ en ppm (DMSO d 6) : 1,17-1,48 (massif, 9H) ; 1,63 (m, 2H) ; 1,82-1,86 (m, 2H) ; 3,36 (m, 1H) ; 4,48 (s, 2H) ; 4,71 (m, 1H) ; 7,34-7,74 (massif, 8H) ; 9,46 (d, 1H) ; 12,48 (s, 1H).
$[\alpha]_D^{20}$ = -122,9 (c = 1/$CH_3OH$).

**Exemple 5 :** (2S)-N-(5-{2-[(cyclohexyloxy)méthyl)phényl}-1,3-thiazol-2-yl)-2-{[2-(3,5-difluorophényl)propanoyl]amino}pentanamide (composé n°58).

On procède de la même manière que dans l'étape 1.8 de l'exemple 1 en faisant réagir le chlorhydrate de (2S)-2-amino-*N*-(5-{2-[(cyclohexyloxy)méthyl]phényl}-1,3-thiazol-2-yl)pentanamide, obtenu par une méthode similaire à celle décrite pour la préparation du chlorhydrate de (2S)-2-amino-*N*-(5-{2-[(cyclohexyloxy)méthyl]phényl}-1,3-thiazol-2-yl)propionamide (étapes 1.1 à 1.7 de l'exemple 1), avec l'acide 2-(3,5-difluorophényl)propanoïque. On obtient 0,52g du composé. F= 88°C.
RMN $^{1H}$ δ en ppm (DMSO d 6) : 0,82 (t, 3H) ; 1,18-1,91 (massif, 17H) ; 2,51 (m, 1H) ; 3,82 (q, 1H) ; 4,45 (s, 2H) ; 4,49 (m, 1H) ; 7,01-7,09 (massif, 3H) ; 7,36-7,64 (massif, 5H) ; 8,44 (d, 1H) ; 12,30 (s, 1H).
$[\alpha]_D^{20}$ = -78 (c = 1/$CH_3OH$).

**Exemple 6 :** (2S)-2-{[2-(3,5-difluorophényl)acétyl]amino}-*N*-(5-{2-(isopropoxyméthyl)-4-méthoxyphényl}-1,3-thiazol-2-yl)pentanamide (composé n°75).

**Exemple 6.1** : 2-bromo-5-méthoxy-benzol.

Le 2-bromo-5-méthoxy-benzol est préparé à partir du 2-bromo-5-méthoxybenzoate de méthyle, disponible dans le commerce, selon une méthode décrite par Stara, Irena G. et al., Tetrahedron, 54, 37, 1998, 11209-11234.

**Exemple 6.2 :** bromure de 2-bromo-5-méthoxy-benzyle.

Le bromure de 2-bromo-5-méthoxy-benzyle est préparé à partir du 2-bromo-5-méthoxy-benzol, obtenu à l'étape 6.1, selon une méthode décrite par Fukuyama, Yoshiyasu et al., Heterocycles, 54, 1, 2001, 259-274.

**Exemple 6.3 :** (2S)-2-{[2-(3,5-difluorophényl)acétyl]amino}-*N*-(5-{2-(isopropoxyméthyl)-4-méthoxyphényl}-1,3-thiazol-2-yl)pentanamide.

Le (2S)-2-{[2-(3,5-difluorophényl)acétyl]amino}-N-(5-{2-(isopropoxyméthyl)-4-méthoxyphényl}-1,3-thiazol-2-yl)pentanamide est préparé à partir du bromure de 2-bromo-5-méthoxy-benzyle, obtenu à l'étape 6.2, selon une méthode analogue à celle décrite aux étapes 1.1 à 1.8 de l'exemple 1.
F= 73,9°C.
RMN $^{1H}$ δ en ppm (DMSO d 6) : 0,87 (t, 3H) ; 1, 12 (d, 6H) ; 1,26-1,40 (m, 2H) ; 1,60-1,72 (m, 2H) ; 3,52 (s, 2H) ; 3,58 (m, 1H) ; 3,72 (s, 3H) ; 4,41 (s, 2H) ; 4,51 (m, 1H) ; 6,92-7,34 (massif, 6H) ; 7,46 (s, 1H) ; 8,52 (d, 1H) ; 12,26 (s, 1H).
$[\alpha]_D^{20}$ = -121 (c = 1/$CH_3OH$).

Les tableaux suivant illustrent les structures chimiques et les propriétés physiques de quelques composés de l'invention.
Dans ces tableaux :

- F(°C) représente le point de fusion du composé en degré Celsius ;
- $[\alpha_D]$ (c=1, $CH_3OH$) représente le pouvoir rotatoire du composé à la concentration de 1 g/l dans le méthanol ;
- (S) ou (R) dans les colonnes « $R_3$ » et « $R_5$, $R_{5'}$ » indiquent la stéréochimie des carbones asymétriques, portant respectivement $R_3$ ou $R_5$, dans la formule (I). Pour le carbone portant $R_5$, l'indication (S) ou (R) ne concerne pas

le cas où $R_5$ et $R_{5'}$ forment ensemble un groupe oxo.

Les composés décrits dans ce tableau sont à l'état de bases, sauf le composé n° 68 qui est sous forme de sel d'hexafluorophosphate. Ils ont été préparés selon les méthodes décrites précédemment.

## Tableau 1

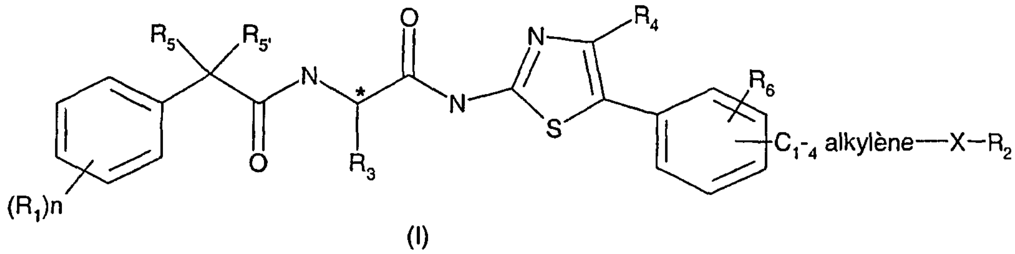

(I)

| N° | $(R_1)n$ | $R_3$ | $R_4$ | $R_5$, $R_{5'}$ | 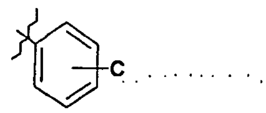 | F (°C) | $[\alpha_D]$ (c=1, $CH_3OH$) |
|---|---|---|---|---|---|---|---|
| 1. | 3-F, 5-F | $-CH_3$ (RS) | H | H, H | 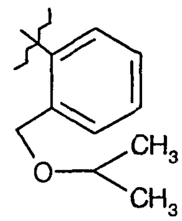 | 175 | - |
| 2. | 3-F, 5-F | $-CH_3$ (RS) | H | H, H | 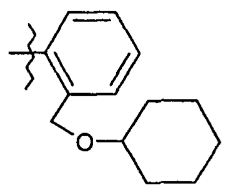 | 126 | - |
| 3. | 3-F, 5-F | $-CH_3$ (RS) | H | H, H | 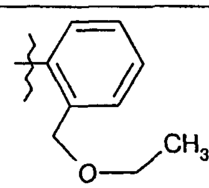 | 165 | - |
| 4. | 3-F, 5-F | $-CH_3$ (RS) | H | H, H | 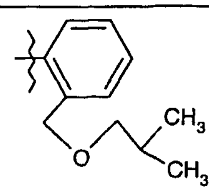 | 181 | - |
| 5. | 3-F, 5-F | $-CH_3$ (RS) | H | H, H | 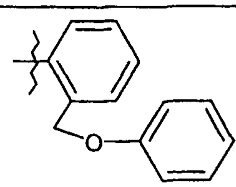 | 114 | - |
| 6. | 3-F, 5-F | $-CH_3$ (RS) | H | H, H | 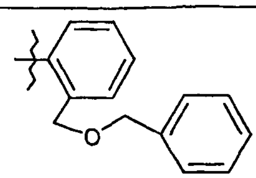 | 147 | - |

| N° | $(R_1)n$ | $R_3$ | $R_4$ | $R_5$, $R_{5'}$ | 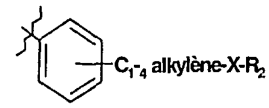 | F (°C) | [$\alpha_D$] (c=1, $CH_3OH$) |
|---|---|---|---|---|---|---|---|
| 7. | 3-F, 5-F | $-CH_3$ (RS) | H | H, H | 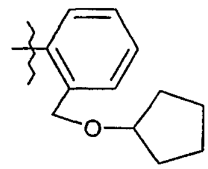 | 146 | - |
| 8. | 3-F, 5-F | $-CH_3$ (RS) | H | H, H | 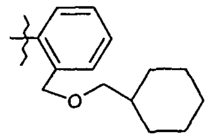 | 143 | - |
| 9. | 3-F, 5-F | $-CH_2CH_3$(RS) | H | H, H | 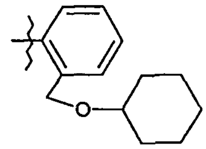 | 179 | - |
| 10. | 3-F, 5-F | $-CH(CH_3)_2$(RS) | H | H, H | 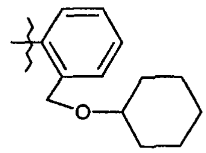 | 181 | - |
| 11. | n=0 | $-CH_3$ (RS) | H | H, H | 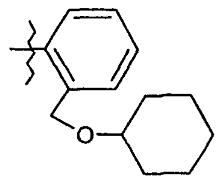 | 162 | - |
| 12. | 3-F, 5-F | $-CH_3$ (RS) | $-CH_3$ | H, H | 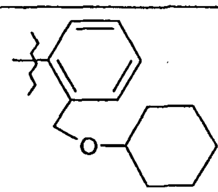 | 152 | - |
| 13. | 3-F, 5-F | $-CH_3$ (RS) | H | H, H | 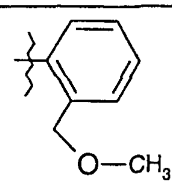 | 194 | - |
| 14. | 3-F, 5-F | $-CH_3$ (RS) | $-CH_3$ | H, H | 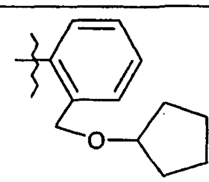 | 104 | - |
| 15. | 3-F, 5-F | H | H | H, H | 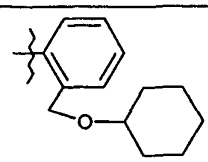 | 172 | - |

| N° | $(R_1)n$ | $R_3$ | $R_4$ | $R_5$, $R_{5'}$ | 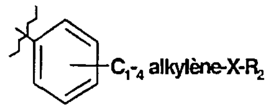 | F (°C) | $[\alpha_D]$ (c=1, $CH_3OH$) |
|---|---|---|---|---|---|---|---|
| 16. | 3-$CH_3$ | -$CH_3$ (S) | H | H, H | 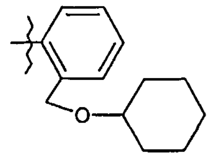 | 98 | -125 |
| 17. | 4-$CH_3$ | -$CH_3$ (S) | H | H, H | 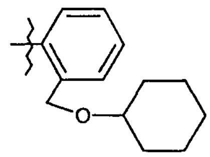 | 239 | -132 |
| 18. | 3-Cl | -$CH_3$ (S) | H | H, H | 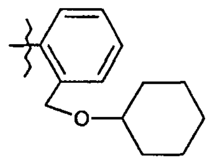 | 96 | -128 |
| 19. | 3-F | -$CH_3$ (S) | H | H, H | 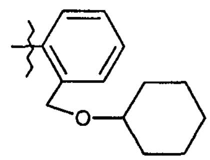 | 98 | -144 |
| 20. | 4-F | -$CH_3$ (S) | H | H, H | 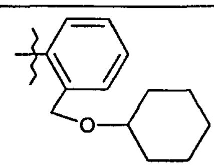 | 70 | -128 |
| 21. | 3-F, 5-F | -$CH_3$ (S) | H | H, H | 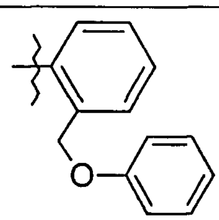 | 78 | -141 |
| 22. | 3-F, 5-F | -$CH_3$ (S) | H | H, H | 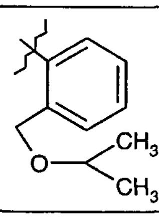 | 136 | -137 |
| 23. | 3-F, 5-F | -$CH_3$ (S) | -$CH_3$ | H, H | 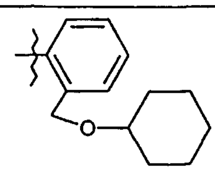 | 85 | -52 |

| N° | $(R_1)n$ | $R_3$ | $R_4$ | $R_5$, $R_{5'}$ | 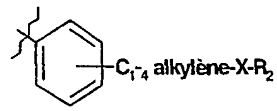 | F (°C) | $[\alpha_D]$ (c=1, $CH_3OH$) |
|---|---|---|---|---|---|---|---|
| 24. | 3-F, 5-F | $-CH_3$ (S) | H | H, H | 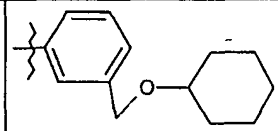 | 123 | -190 |
| 25. | 3-F, 5-F | $-CH_3$ (R) | H | H, H | 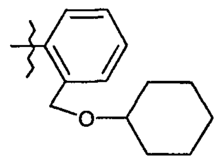 | 95 | +135,8 |
| 26. | 3-F, 5-F | $-CH_3$ (S) | H | H, H | 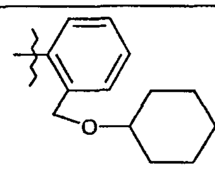 | 140 | -138,6 |
| 27. | 3-F, 5-F | $-CH_3$ (S) | $-CH_3$ | H, H | 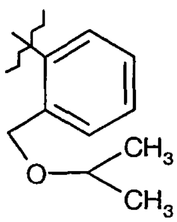 | 74 | -117,8 |
| 28. | 3-F, 5-F | $-CH_3$ (S) | $-CH_3$ | H, H | 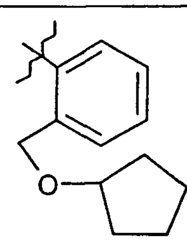 | 141 | -93,5 |
| 29. | 3,4 (-O-$CH_2$-O-) | $-CH_3$ (S) | H | H, H | 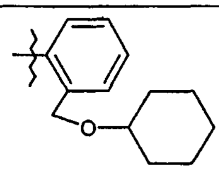 | 11 | -124 |
| 30. | 3-F, 4-F | $-CH_3$ (S) | H | H, H | 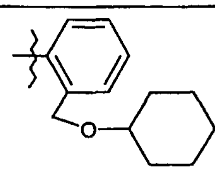 | 136 | -132 |
| 31. | 3-F, 5-F | $-CH(CH_3)_2$ (S) | H | H, H | 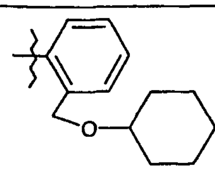 | 87 | -106 |

| N° | $(R_1)n$ | $R_3$ | $R_4$ | $R_5$, $R_{5'}$ | 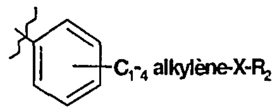 | F (°C) | [$\alpha_D$] (c=1, $CH_3OH$) |
|---|---|---|---|---|---|---|---|
| 32. | 3-F, 5-F | $-CH_2CH_3$ (S) | H | H, H | 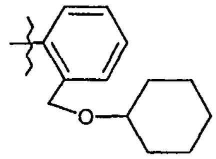 | 68 | -122 |
| 33. | 4-$OCH_3$ | $-CH_3$ (S) | H | H, H | 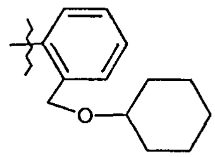 | 74 | -149 |
| 34. | 3-$OCH_3$ | $-CH_3$ (S) | H | H, H | 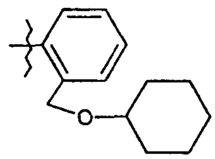 | 94 | -139 |
| 35. | 3-$OCH_3$, 5-$OCH_3$ | $-CH_3$ (S) | H | H, H | 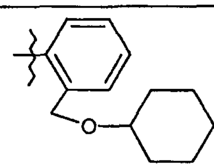 | 73 | -131 |
| 36. | 3-F, 5-F | $-CH_2CH(CH_3)_2$ (S) | H | H, H | 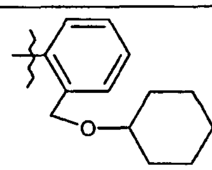 | 67 | -93 |
| 37. | 3-F, 5-F | $-C(CH_3)_3$ (S) | H | H, H | 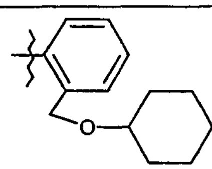 | 73 | -107 |
| 38. | 4-Cl | $-CH_3$ (S) | H | H, H | 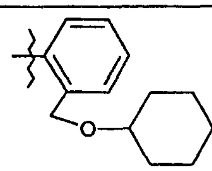 | 125 | -149,4 |
| 39. | 3-F , 5-F | $-CH_3$ (S) | H | H, H | 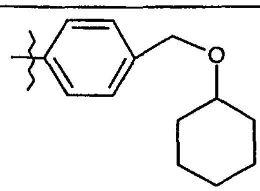 | 155 | -162 |
| 40. | 3-F , 5-F | $-CH_3$ (S) | H | H, H | 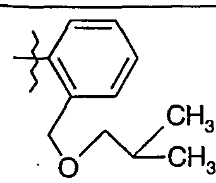 | 161 | -139,1 |

| N° | $(R_1)n$ | $R_3$ | $R_4$ | $R_5$, $R_{5'}$ | 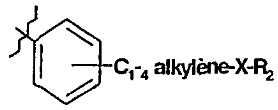 | F (°C) | $[\alpha_D]$ (c=1, $CH_3OH$) |
|---|---|---|---|---|---|---|---|
| 41. | 3-F, 5-F | $-CH_3$ (S) | H | H, H | 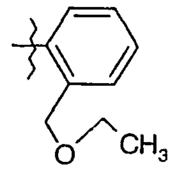 | 173 | -149 |
| 42. | 3-F, 5-F | $-(CH_2)_2CH_3$ (S) | H | H, H | 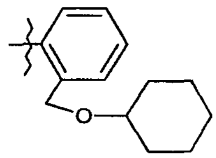 | 134 | -102 |
| 43. | 3-F, 5-F | $-CH_3$ (S) | H | H, H | 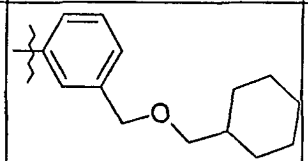 | 144 | -165 |
| 44. | 3-F, 5-F | 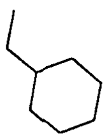 (S) | H | H, H | 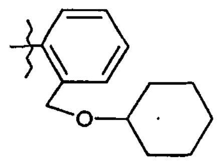 | 120 | -56 |
| 45. | 3-F, 5-F | $-CH_3$ (S) | H | H, H | 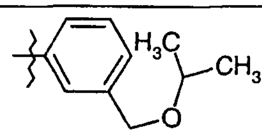 | 136 | -121 |
| 46. | 3-F, 5-F | $-CH_3$ (S) | H | H, H | 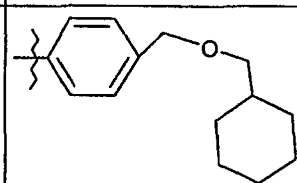 | 166 | -166,6 |
| 47. | 3-F, 5-F | $-(CH_2)_3CH_3$ (S) | H | H, H | 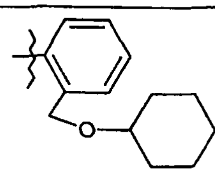 | 77,6 | -84 |
| 48. | 3-$OCF_3$ | $-CH_3$ (S) | H | H, H | 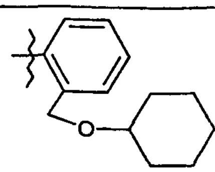 | 60 | -119 |

| N° | $(R_1)n$ | $R_3$ | $R_4$ | $R_5$, $R_{5'}$ | 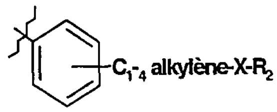 | F (°C) | $[\alpha_D]$ (c=1, $CH_3OH$) |
|---|---|---|---|---|---|---|---|
| 49. | 3-F, 5-F | $-CH_3$ (S) | H | H, H | 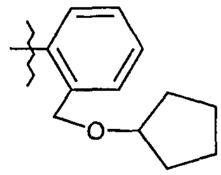 | 236 | -140,3 |
| 50. | 3-F, 5-F | $-CH_3$ (S) | H | OH, H ( R) | 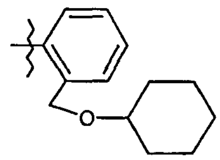 | 169 | -106 |
| 51. | 3-F, 5-F | $-CH_3$ (S) | H | OH, H ( S) | 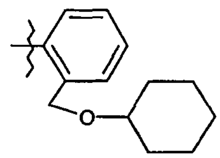 | 159 | -95 |
| 52. | 3-F, 5-F | $-(CH_2)_2CH_3$ (S) | $-CH_3$ | H, H | 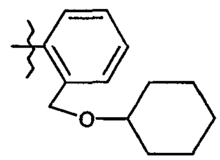 | 94 | -88,3 |
| 53. | 3-F, 5-F | $-(CH_2)_2CH_3$ (S) | $-CH_3$ | OH, H (R) | 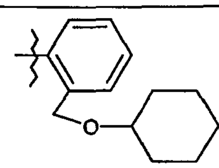 | 99,4 | -101,9 |
| 54. | 3-F, 5-F | $-CH_3$ (S) | H | H, H | 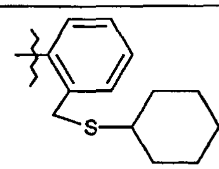 | 91,6 | -116,5 |
| 55. | 3-F, 5-F | $-(CH_2)_2CH_3$ (S) | H | OH, H ( S) | 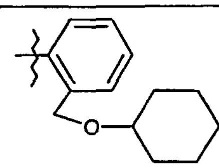 | 141 | -74,5 |
| 56. | 3-F, 5-F | $-(CH_2)_2CH_3$ (S) | H | OH, H ( R) | 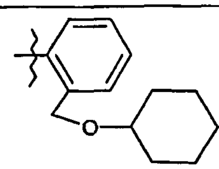 | 112 | -64,2 |
| 57. | 3-F, 5-F | $-(CH_2)_2CH_3$ (S) | $-CH_3$ | OH, H (S) | 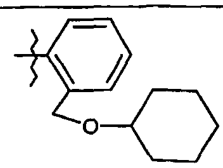 | 115 | -63,2 |

| N° | $(R_1)n$ | $R_3$ | $R_4$ | $R_5$, $R_{5'}$ | 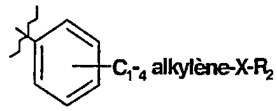 | F (°C) | $[\alpha_D]$ (c=1, $CH_3OH$) |
|---|---|---|---|---|---|---|---|
| 58. | 3-F, 5-F | $-(CH_2)_2CH_3$ (S) | H | $CH_3$, H | 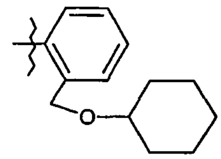 | 88 | -78 |
| 59. | 3-F, 5-F | $-(CH_2)_2CH_3$ (S) | $-CH_2CH_3$ | H, H | 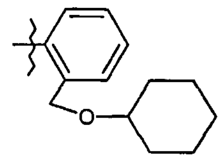 | 80 | -94,9 |
| 60. | 3-F , 5-F | $-(CH_2)_2CH_3$ (S) | H | =O | 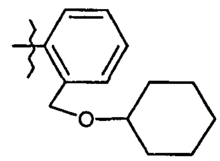 | 77 | -94,5 |
| 61. | 3-F , 5-F | $-CH_3$ (S) | H | =O | 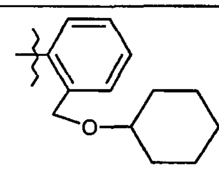 | 78 | -122,9 |
| 62. | 3-F , 5-F | $-CH_3$ (S) | $-CH_2CH_3$ | H, H | 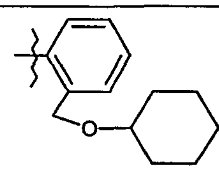 | 70 | -118 |
| 63. | 3-F , 5-F | $-(CH_2)_2CH_3$ (S) | $CH(CH_3)_2$ | H, H | 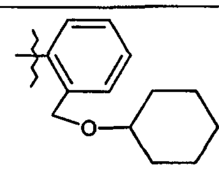 | 74 | -81,6 |
| 64. | 3-F , 5-F | $-(CH_2)_2CH_3$ (S) | H | F, H | 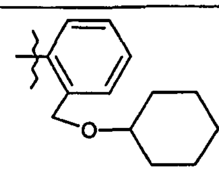 | 139 | -109 |
| 65. | 3-F , 5-F | $-CH_3$ (S) | H | F, H ( R) | 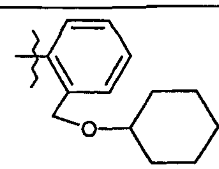 | 78 | -115,4 |
| 66. | 3-F , 5-F | $-CH_3$ (S) | H | F, H (S) | 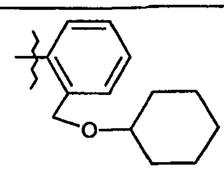 | 158 | -92 |

| N° | $(R_1)n$ | $R_3$ | $R_4$ | $R_5$, $R_{5'}$ | 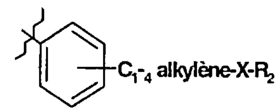 | F (°C) | $[\alpha_D]$ (c=1, $CH_3OH$) |
|---|---|---|---|---|---|---|---|
| 67. | 3-F , 5-F | $-(CH_2)_2CH_3$ (S) | H | F, H | 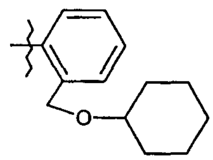 | 91 | -91 |
| 68. | 3-F , 5-F | $-(CH_2)_2CH_3$ (S) | $-CH_3$ | H, H | 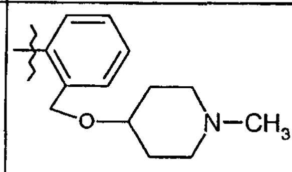 | 186 | -51,6 |
| 69. | 3-F , 5-F | $-CH_2CH_3$ (S) | $-CH_3$ | H, H | 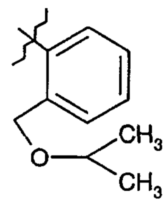 | 79 | -107,8 |
| 70. | 3-F , 5-F | $-CH_2CH_3$ (S) | H | H, H | 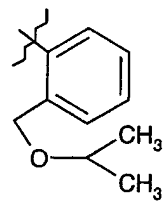 | 73 | -119,8 |
| 71. | 3-F , 5-F | $-(CH_2)_2CH_3$ (S) | $-CH_3$ | H, H | 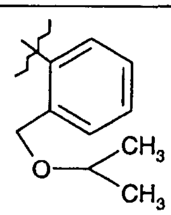 | 66,6 | -93,5 |
| 72. | 3-F , 5-F | $-(CH_2)_2CH_3$ (S) | H | H, H | 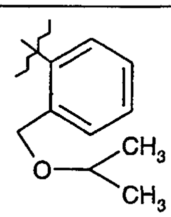 | 74 | -114 |
| 73. | 3-F , 5-F | $-CH_3$ (S) | H | H, H | 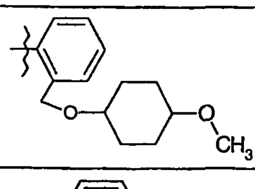 | 149 | -115 |
| 74. | 3-F , 5-F | $-(CH_2)_2CH_3$ (S) | H | F, F | 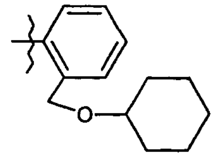 | 68 | -66,2 |

| N° | $(R_1)n$ | $R_3$ | $R_4$ | $R_5$, $R_{5'}$ | 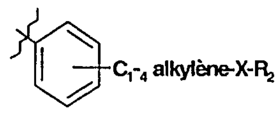 | F (°C) | $[\alpha_D]$ (c=1, $CH_3OH$) |
|---|---|---|---|---|---|---|---|
| 75. | 3-F , 5-F | $-(CH_2)_2CH_3$ (S) | H | H, H | 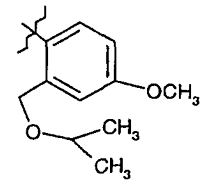 | 73,9 | -121 |

Les composés de l'invention ont fait l'objet d'essais pharmacologiques qui ont montré leur intérêt comme substances actives en thérapeutique.

Ils ont en particulier été testés quant à leurs effets d'inhibition de la production du peptide β-amyloïde (β-A4).

Le peptide β-amyloïde (β-A4) est un fragment d'une protéine précurseur plus importante appelée APP (Amyloid Precursor Protein). Cette dernière est produite et présente dans différentes cellules de tissu animal ou humain. Au niveau cérébral, son clivage par des enzymes de type protéase conduit à la formation du peptide β-A4 qui s'accumule sous forme de plaque amyloïde. Les deux protéases responsables de la production du peptide amyloide sont connues sous le nom de beta et gamma-secrétases (Wolfe MS, Secretase targets for Alzheimer's disease: identification and therapeutic potential, J. Med. Chem., 2001 Jun 21;44(13), 2039-60).

Or il a été démontré que ce dépôt progressif du peptide β-A4 est neurotoxique et pourrait jouer un rôle important dans la maladie d'Alzheimer.

Ainsi, les composés de la présente invention, en tant qu'inhibiteur de la production du peptide β-amyloïde (β-A4) par inhibition de la gamma protéase, peuvent être utilisés dans le traitement de pathologies comme la démence sénile, la maladie d'Alzheimer, le syndrome de Down, la maladie de Parkinson, l'angiopathie amyloïde et/ou les désordres cérébro-vasculaires.

Les tests ont été réalisés selon le protocole décrit ci-après.

Pour l'essai cellulaire β amyloïde, la lignée CHO-K1 coexprimant le CT100 de APP et PS1 M146L clone 30-12 est utilisée. La lignée cible l'inhibition de gamma secrétase. La préséniline est liée à l'activité gamma-secrétase (Wolfe MS, Haass C., The Role of presenilins in gamma-secretase activity, J. Biol. Chem., 2001 Feb 23, 276(8), 5413-6) et sa coexpression avec la protéine amyloïde ou son fragment N-terminal entraîne une augmentation de secrétion du peptide A1-42 (β-A4) générant ainsi un outil pharmacologique permettant d'évaluer l'inhibition par les composés de formule (I) de la production du peptide β-A4. L'ensemencement des plaques de culture de 96 puits est réalisé à raison de $1x10^5$ cellules par puits dans 150µl de milieu d'incubation. La présence d'un pourcentage minimal (1,3% final) de sérum permet l'adhésion cellulaire au plastique après 2-3 heures d'incubation à 37°C, en présence de 5% $CO_2$. Les produits (15µl) sont testés à 10µM DMSO 1% final et sont incubés durant 24-25h à 37°C en présence de 5% $CO_2$ et de 100% d'humidité. Après cette incubation de 24-25h, les surnageants cellulaires (100µl) sont transférés dans les plaques ELISA, traitées avec l'anticorps de capture 6E10 (6E10, épitope : aa1-17, INTERCHIM/SENETEK 320-10), pour déterminer le taux de peptides amyloïdes sécrété par les cellules en présence de composés selon l'invention. Une gamme de peptide contrôle, « peptide 1-40 », synthétique à 5 et 10 ng/ml est traitée parallèlement. Les plaques ELISA sont incubées pendant une nuit à 4°C.

La quantité de peptide fixé est détectée de façon indirecte en présence d'un compétiteur correspondant au peptide tronqué, le peptide 1-28 couplé à la biotine qui est ensuite détectée par la streptavidine couplée à la phosphatase alcaline. Le substrat, le p-Nitrophényl Phosphate (pNPP *FAST* p-Nitrophényl Phosphate, Sigma N2770), donne un produit de réaction soluble jaune lisible à 405nm. La réaction est stoppée avec une solution d'EDTA 0,1M. Pour cela, après fixation du peptide amyloïde dans la plaque ELISA, 50µl de peptide 1-28 biotinylé sont ajoutés aux 100µl de surnageant cellulaire et incubés 30 minutes à température ambiante. Les plaques ELISA sont ensuite lavées 3 fois. Après séchage par inversion sur papier absorbant, 100µl de streptavidine-Alcaline Phosphatase (Interchim/Jackson ImmunoResearch Laboratories 016-050-084), sont ajoutés par puits et incubés 1 heure à température ambiante. Les plaques sont à nouveau lavées puis le substrat de la phosphatase alcaline (pNPP 1mg/ml) est ajouté à raison de 100µl par puits. Après une incubation de 30 minutes à température ambiante, la réaction est stoppée par ajout de 100µl par puits d'EDTA 0,1M et la lecture est effectuée à 405nm.

Les composés de formule (I) selon l'invention ont montré une CI50 (concentration inhibitrice à 50%) inférieure à 500 nM, plus particulièrement inférieure à 100 nM.

Les résultats des tests biologiques montrent que les composés sont des inhibiteurs de la formation du peptide

β-amyloïde (β-A4).

Ainsi, ces composés peuvent être employés dans le traitement des pathologies dans lesquelles un inhibiteur de la formation du peptide β-amyloïde (β-A4) apporte un bénéfice thérapeutique. Notamment de telles pathologies sont la démence sénile, la maladie d'Alzheimer, le syndrome de Down, la maladie de Parkinson, l'angiopathie amyloïde et les désordres cérébro-vasculaires.

L'utilisation des composés selon l'invention pour la préparation d'un médicament destiné à traiter les pathologies ci-dessus mentionnées fait partie intégrante de l'invention.

L'invention a également pour objet des médicaments qui comprennent un composé de formule (1), ou un sel d'addition de ce dernier à un acide pharmaceutiquement acceptable ou encore un hydrate ou un solvat du composé de formule (I). Ces médicaments trouvent leur emploi en thérapeutique, notamment dans le traitement des pathologies ci-dessus mentionnées.

Selon un autre de ses aspects, la présente invention concerne des compositions pharmaceutiques renfermant en tant que principe actif, au moins un composé selon l'invention. Ces compositions pharmaceutiques contiennent une dose efficace d'un composé selon l'invention, ou un sel pharmaceutiquement acceptable, un hydrate ou un solvat dudit composé, et éventuellement un ou plusieurs excipients pharmaceutiquement acceptables.
Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intraveineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules, les chewing-gums et les solutions ou suspensions orales, les formes d'administration sublinguale, buccale, intratrachéale, inraoculaire, intranasale, par inhalation, les formes d'administration sous-cutanée, intramusculaire ou intraveineuse et les formes d'administration rectale ou vaginale. Pour l'application topique, on peut utiliser les composés selon l'invention dans des crèmes, pommades ou lotions.

Par exemple, lorsqu'on prépare une composition solide sous forme de comprimés, on mélange l'ingrédient actif principal avec un excipient pharmaceutique, tel que la gélatine, l'amidon, le lactose, le stéarate de magnésium, le talc, la gomme arabique ou analogues. On peut enrober les comprimés de saccharose, d'un dérivé cellulosique, ou d'autres matières. Les comprimés peuvent être réalisés par différentes techniques, compression directe, granulation sèche, granulation humide ou fusion à chaud.

Afin d'obtenir l'effet prophylactique ou thérapeutique désiré, la dose de principe actif peut varier entre 0,1 mg et 200 mg par kg de poids du corps et par jour. Bien que ces dosages soient des exemples de situation moyenne, il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, le poids et la réponse dudit patient.

Chaque dose unitaire peut contenir de 0,1 à 1000 mg, de préférence de 0,1 à 500 mg, de principe actif en combinaison avec un ou plusieurs excipients pharmaceutiques. Cette dose unitaire peut être administrée 1 à 5 fois par jour de façon à administrer un dosage journalier de 0,5 à 5000 mg, de préférence de 0,5 à 2500 mg.

La présente invention selon un autre de ses aspects, concerne également une méthode de traitement des pathologies ci-dessus indiquées qui comprend l'administration d'un composé selon l'invention, d'un sel pharmaceutiquement acceptable, d'un solvat ou d'un hydrate dudit composé.

## Revendications

1. Composé répondant à la formule générale (1) :

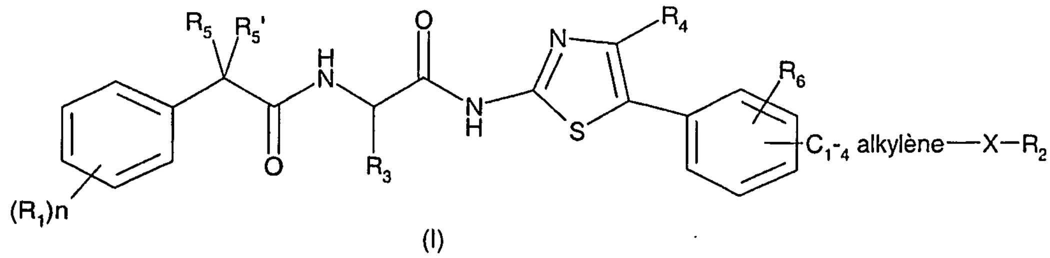

(I)

dans laquelle,
n est égal à 0, 1, 2 ou 3 ;
X représente un atome d'oxygène ou de soufre ;
$R_1$ représente, indépendamment l'un de l'autre lorsque n = 2 ou 3, un atome d'halogène, un hydroxy, un $C_{1-3}$ alkyle, un $C_{1-3}$ alcoxy, un trifluorométhyle, un trifluorométhyloxy ou un méthylènedioxy ;
$R_2$ représente un groupe $C_{1-6}$ alkyle éventuellement substitué par un groupe $C_{3-7}$ cycloalkyle, un phényle, un groupe $C_{1-3}$ alcoxy, un hydroxy ou un atome d'halogène; un groupe $C_{3-7}$ cycloalkyle, pipéridinyle ou phényle ;
les groupes $C_{3-7}$ cycloalkyle, pipéridinyle et phényle étant éventuellement substitués par un ou plusieurs groupes $C_{1-3}$ alkyle, $C_{1-3}$ alcoxy, un hydroxy ou un atome d'halogène ;
$R_3$ représente un atome d'hydrogène ou un groupe $C_{1-6}$ alkyle éventuellement substitué par un groupe $C_{3-7}$ cycloalkyle ;
$R_4$ représente un atome d'hydrogène ou un groupe $C_{1-4}$ alkyle ;
$R_5$ et $R_{5'}$ représentent, indépendamment l'un de l'autre, un atome d' hydrogène, un hydroxy, un atome d'halogène, un groupe $C_{1-3}$ alkyle; ou $R_5$ et $R_{5'}$ forment ensemble un groupe oxo ; et
$R_6$ représente un atome d'hydrogène, un atome d'halogène, un $C_{1-3}$alkyle, un $C_{1-3}$alcoxy, un trifluorométhyle, ou un trifluorométhoxy ;
à l'état de base, de sel d'addition à un acide, d'hydrate ou de solvat.

2. Composé selon la revendication 1, **caractérisé en ce que** :

X représente un atome d'oxygène ou de soufre;
$R_1$ représente, indépendamment l'un de l'autre lorsque n = 2 ou 3, un atome d'halogène, un méthylènedioxy, un groupe $C_{1-3}$ alkyle, $C_{1-3}$ alcoxy ou un trifluorométhyloxy ;
$R_2$ représente un groupe $C_{1-4}$ alkyle éventuellement substitué par un groupe $C_{4-7}$ cycloalkyle ou phényle ; un groupe $C_{4-7}$ cycloalkyle, pipéridinyle ou phényle ; les groupes $C_{4-7}$ cycloalkyle, pipéridinyle et phényle étant éventuellement substitués par un ou plusieurs groupes $C_{1-3}$ alkyle ou $C_{1-3}$ alcoxy ;
$R_3$ représente un atome d'hydrogène ou un groupe $C_{1-4}$ alkyle éventuellement substitué par un groupe $C_{4-7}$ cycloalkyle;
$R_4$ représente un atome d'hydrogène ou un groupe $C_{1-3}$ alkyle ;
$R_5$ et $R_{5'}$ représentent, indépendamment l'un de l'autre, un atome d'hydrogène, d'halogène, un hydroxy ou un groupe $C_{1-3}$ alkyle ; ou $R_5$ et $R_{5'}$ forment ensemble un groupe oxo ; et
$R_6$ représente un atome d'hydrogène ou un $C_{1-3}$alcoxy ;

à l'état de base, de sel d'addition à un acide, d'hydrate ou de solvat.

3. Composé de formule (I) selon la revendication 1 **caractérisé en ce que** :

X représente un atome d'oxygène;
$R_1$ représente, indépendamment l'un de l'autre lorsque n = 2 ou 3, un atome d'halogène ;
$R_2$ représente un groupe $C_{1-4}$ alkyle éventuellement substitué par un groupe $C_{4-7}$ cycloalkyle ou phényle ; un groupe $C_{4-7}$ cycloalkyle, pipéridinyle ou phényle ; les groupes $C_{4-7}$ cycloalkyle, pipéridinyle et phényle étant éventuellement substitués par 1 ou 2 groupes $C_{1-3}$ alkyle ou $C_{1-3}$ alcoxy ;
$R_3$ représente un méthyle, éthyle ou propyle ;
$R_4$ représente un atome d'hydrogène ou un méthyle ;
$R_5$ et $R_{5'}$ représentent, indépendamment l'un de l'autre, un atome d'hydrogène, de fluor, un hydroxy ou un méthyle; ou $R_5$ et $R_{5'}$ forment ensemble un groupe oxo ; et
$R_6$ représente un atome d'hydrogène ou un méthoxy ;

à l'état de base, de sel d'addition à un acide, d'hydrate ou de solvat.

4. Procédé de préparation d'un composé de formule (I) selon l'une quelconque des revendications 1 à 3, **caractérisé en ce que** :

on réalise un couplage peptidique du composé de formule (VII)

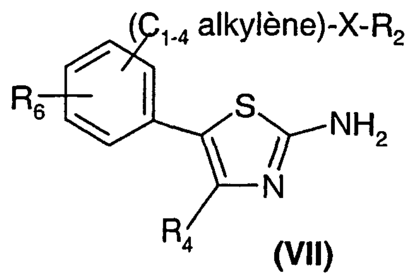

**(VII)**

avec l'aminoacide de formule (VIII)

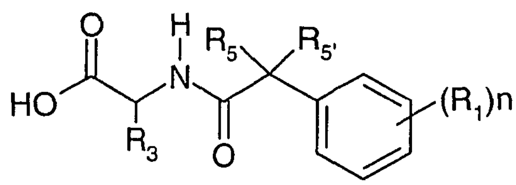

**(VIII)**

dans lesquelles n, X, $R_1$, $R_2$, $R_3$, $R_4$, $R_5$ et $R_{5'}$ sont tels que défini dans la formule (I) selon la revendication 1.

5. Procédé de préparation d'un composé de formule (I) selon l'une quelconque des revendications 1 à 3, dans laquelle $R_3$ ne représente pas un atome hydrogène, par couplage peptidique d'une amine de formule (XIII)

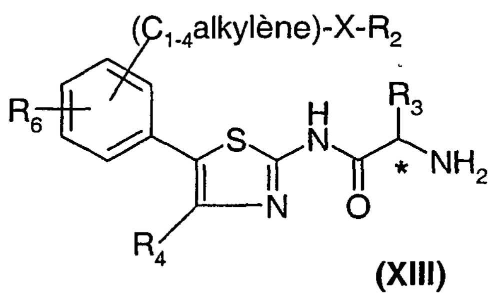

**(XIII)**

avec un acide de formule (XIV)

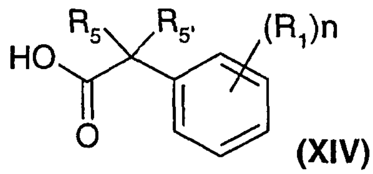

(XIV)

dans lesquelles n, X, $R_1$, $R_2$, $R_3$, $R_4$, $R_5$ et $R_{5'}$ sont tels que défini dans la formule (I) selon la revendication 1.

**6.** Composé de formule (VI)

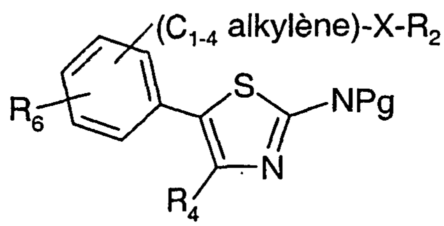

dans laquelle X, $R_2$ et $R_4$ sont tels que définis dans la revendication 1 et Pg représente une diphénylcétone imine.

**7.** Composé de formule (VII)

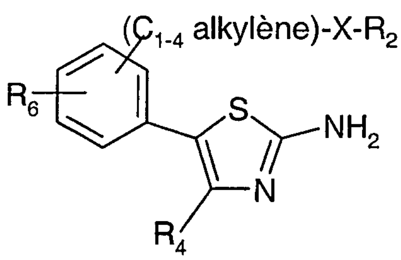

(VII)

dans laquelle X, $R_2$ et $R_4$ sont tels que définis dans la revendication 1.

**8.** Composé de formule (XIII)

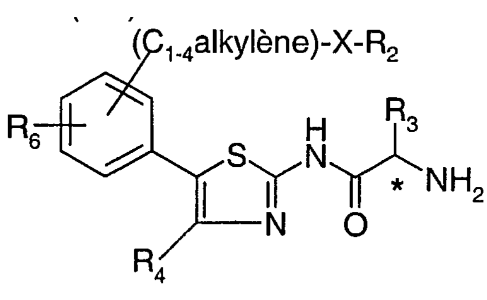

(XIII)

dans laquelle X, $R_2$, $R_3$ et $R_4$ sont tels que définis dans la revendication 1.

**9.** Composition pharmaceutique contenant au moins un composé de formule (I) selon l'une quelconque des revendications 1 à 3, à l'état de base, de sel, d'hydrate ou de solvat pharmaceutiquement acceptable et éventuellement

un ou plusieurs excipients pharmaceutiquement acceptables.

**10.** Composé de formule (I) selon l'une quelconque des revendications 1 à 3, à l'état de base, de sel, d'hydrate ou de solvat pharmaceutiquement acceptables, pour son utilisation comme médicament.

**11.** Utilisation d'un composé de formule (I) selon l'une quelconque des revendications 1 à 3, à l'état de base, de sel, d'hydrate ou de solvat pharmaceutiquement acceptable, pour la préparation d'un médicament destiné à traiter une pathologie dans laquelle un inhibiteur de la formation du peptide β-amyloïde β-A4 apporte un bénéfice thérapeutique.

**12.** Utilisation d'un composé de formule (I) selon l'une quelconque des revendications 1 à 3, à l'état de base, de sel, d'hydrate ou de solvat pharmaceutiquement acceptable, pour la préparation d'un médicament destiné à traiter la démence sénile, la maladie d'Alzheimer, le syndrome de Down, la maladie de Parkinson, l'angiopathie amyloïde et/ou les désordres cérébro-vasculaires.

## Patentansprüche

**1.** Verbindung, entsprechend der allgemeinen Formel (I)

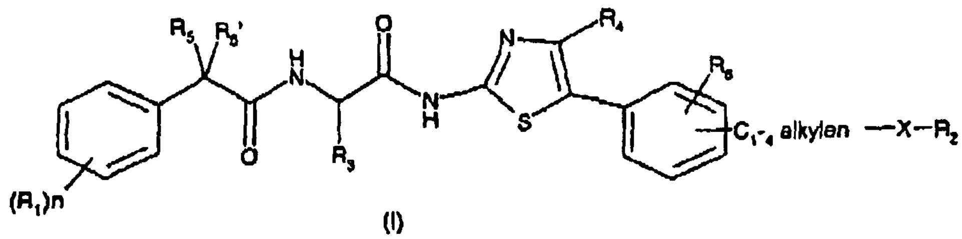

(I)

in der
n gleich 0, 1, 2 oder 3 ist;
X ein Sauerstoffatom oder ein Schwefelatom darstellt;
$R_1$, unabhängig voneinander, wenn n = 2 oder 3 ist, ein Halogenatom, ein Hydroxy, ein $C_{1-3}$-Alkyl, ein $C_{1-3}$-Alkoxy, ein Trifluormethyl, ein Trifluormethoxy oder ein Methylendioxy darstellt;
$R_2$ eine Gruppe $C_{1-6}$-Alkyl, gegebenenfalls substituiert durch eine Gruppe $C_{3-7}$-Cycloalkyl, ein Phenyl, eine Gruppe $C_{1-3}$-Alkoxy, ein Hydroxy oder ein Halogenatom; eine Gruppe $C_{3-7}$-Cycloalkyl, Piperidinyl oder Phenyl darstellt;
wobei die Gruppen $C_{3-7}$-Cycloalkyl, Piperidinyl und Phenyl gegebenenfalls substituiert sind durch eine oder mehrere Gruppen $C_{1-3}$-Alkyl , $C_{1-3}$-Alkoxy, ein Hydroxy oder ein Halogenatom;
$R_3$ ein Wasserstoffatom oder eine Gruppe $C_{1-6}$-Alkyl darstellt, gegebenenfalls substituiert durch eine Gruppe $C_{3-7}$-Cyaloalkyl;
$R_4$ ein Wasserstoffatom oder eine Gruppe $C_{1-4}$-Alkyl darstellt;
$R_5$ und $R_{5'}$, unabhängig voneinander, ein Wasserstoffatom, ein Hydroxy, ein Halogenatom, eine Gruppe $C_{1-3}$-Alkyl darstellen; oder $R_5$ und $R_{5'}$ zusammen eine Gruppe Oxo bilden; und
$R_6$ ein Wasserstoffatom, ein Halogenatom, ein $C_{1-3}$-Alkyl, ein $C_{1-3}$-Alkoxy, ein Trifluormethyl oder ein Trifluormethoxy darstellt;
im Zustand von Base, Additionssalz mit einer Säure, Hydrat oder Solvat.

**2.** Verbindung nach Anspruch 1, **dadurch gekennzeichnet, daß**
X ein Sauerstoffatom oder ein schwefelatom darstellt;
$R_1$, unabhängig voneinander, wenn n = 2 oder 3 ist, ein Halogenatom, ein Methylendioxy, eine Gruppe $C_{1-3}$-Alkyl, $C_{1-3}$-Alkoxy oder ein Trifluormethoxy darstellt;
$R_2$ eine Gruppe $C_{1-4}$-Alkyl, gegebenenfalls substituiert durch eine Gruppe $C_{4-7}$-Cycloalkyl oder Phenyl; eine Gruppe $C_{4-7}$-Cycloalkyl, Piperidinyl oder Phenyl darstellt;
wobei die Gruppen $C_{4-7}$-Cycloalkyl, Piperidinyl und Phenyl gegebenenfalls substituiert sind durch eine oder mehrere Gruppen $C_{1-3}$-Alkyl oder $C_{1-3}$Alkoxy;
$R_3$ ein Wasserstoffatom oder eine Gruppe $C_{1-4}$-Alkyl darstellt, gegebenenfalls substituiert durch eine Gruppe $C_{4-7}$-Cycloalkyl;

$R_4$ ein Wasserstoffatom oder eine Gruppe $C_{1-3}$-Alkyl darstellt;
$R_5$ und $R_{5'}$, unabhängig voneinander, ein Wasserstoffatom, ein Halogenatom, ein Hydroxy oder eine Gruppe $C_{1-2}$-Alkyl darstellen; oder $R_5$ und $R_{5'}$ zusammen eine Gruppe Oxo bilden; und
$R_6$ ein Wasserstoffatom oder ein $C_{2-3}$-Alkoxy darstellt;
im Zustand von Base, Additionssalz mit einer Säure, Hydrat oder Solvat.

3. Verbindung der Formel (I) nach Anspruch 1, **dadurch gekennzeichnet, daß**
X ein Sauerstoffatom darstellt;
$R_1$, unabhängig voneinander, wenn n = 2 oder 3 ist, ein Halogenatom darstellt;
$R_2$ eine Gruppe $C_{1-4}$-Alkyl, gegebenenfalls substituiert durch eine Gruppe $C_{4-7}$-Cycloalkyl oder Phenyl; eine Gruppe $C_{4-7}$-Cycloalkyl, Piperidinyl oder Phenyl darstellt;
wobei die Gruppen $C_{4-7}$-Cycloalkyl, Piperidinyl und Phenyl gegebenenfalls substituiert sind durch ein oder zwei Gruppen $C_{1-3}$-Alkyl oder $C_{1-3}$-Alkoxy;
$R_3$ ein Methyl, Ethyl oder Propyl darstellt;
$R_4$ ein Wasserstoffatom oder ein Methyl darstellt;
$R_5$ und $R_{5'}$, unabhängig voneinander, ein Wasserstoffatom, ein Fluoratom, ein Hydroxy oder ein Methyl darstellen; oder $R_5$ und $R_{5'}$ zusammen eine Gruppe Oxo bilden; und
$R_6$ ein Wasserstoffatom oder ein Methoxy darstellt;
im Zustand von Base, Additionssalz mit einer Säure, Hydrat oder Solvat.

4. Verfahren zur Herstellung einer Verbindung der Formel (I) nach irgendeinem der Ansprüche 1 bis 3, **dadurch gekennzeichnet, daß** man eine peptidische Kopplung der Verbindung der Formel (VII)

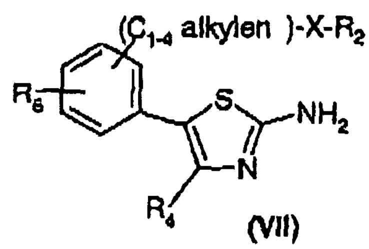

(VII)

mit der Aminosäure der Formel (VIII)

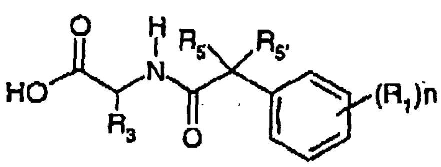

(VIII)

durchführt,
in denen n, X, $R_1$, $R_2$, $R_3$, $R_4$, $R_5$ und $R_{5'}$ wie in der Formel (I) nach Anspruch 1 definiert sind.

5. Verfahren zur Herstellung einer Verbindung der Formel (I) nach irgendeinem der Ansprüche 1 bis 3, worin $R_3$ kein Wasserstoffatom darstellt, durch peptidische Kopplung eines Amins der Formel (XIII)

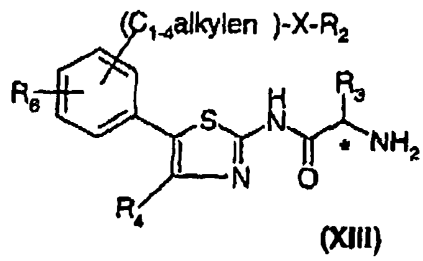

(XIII)

mit einer Säure der Formel (XIV)

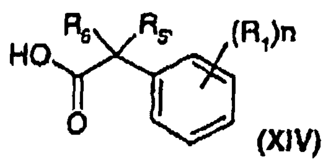

(XIV)

in denen n, X, $R_1$, $R_2$, $R_3$, $R_4$, $R_5$ und $R_{5'}$ wie in der Formel (I) nach Anspruch 1 definiert sind.

**6.** Verbindung der Formel (VI)

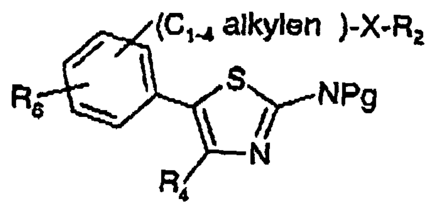

in der X, $R_2$ und $R_4$ wie in Anspruch 1 definiert sind und Pg ein Diphenylketonimin darstellt.

**7.** Verbindung der Formel (VII)

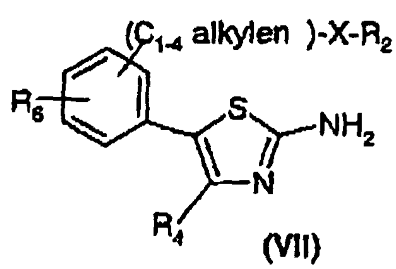

(VII)

in der X, $R_2$ und $R_4$ wie in Anspruch 1 definiert sind.

**8.** Verbindung der Formel (XIII)

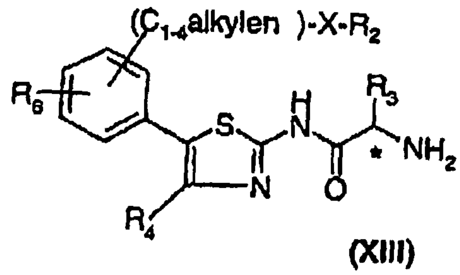

(XIII)

in der X, $R_2$, $R_3$ und $R_4$ wie in Anspruch 1 definiert sind.

9. Pharmazeutische Zusammensetzung, enthaltend mindestens eine Verbindung der Formel (I) nach irgendeinem der Ansprüche 1 bis 3, im Zustand von pharmazeutisch akzeptabler Base, Salz, Hydrat oder Solvat und gegebenenfalls einen oder mehrere pharmazeutisch akzeptable Trägerstoffe.

10. Verbindung der Formel (I) nach irgendeinem der Ansprüche 1 bis 3 im Zustand von pharmazeutisch akzeptabler Base, salz, Hydrat oder Solvat für ihre Anwendung als Arzneimittel.

11. Verwendung einer Verbindung der Formel (I) nach irgendeinem der Ansprüche 1 bis 3 im Zustand von pharmazeutisch akzeptabler Base, Salz, Hydrat oder Solvat zur Herstellung eines Arzneimittels, vorgesehen für die Behandlung einer Pathologie, bei der ein Inhibitor der Bildung des Peptides β-Amyloid β-A4 einen therapeutischen Nutzen einbringt.

12. Verwendung einer Verbindung der Formel (I) nach irgendeinem der Ansprüche 1 bis 3 im Zustand von pharmazeutisch akzeptabler Base, Salz, Hydrat oder Solvat zur Herstellung eines Arzneimittels, vorgesehen für die Behandlung der senilen Demenz, der Alzheimer-Erkrankung, des Down-Syndroms, der Parkinson-Erkrankung, der amyloiden Angiopathie und/oder von cerebrovasculären Störungen.

**Claims**

1. A compound corresponding to the general formula (I):

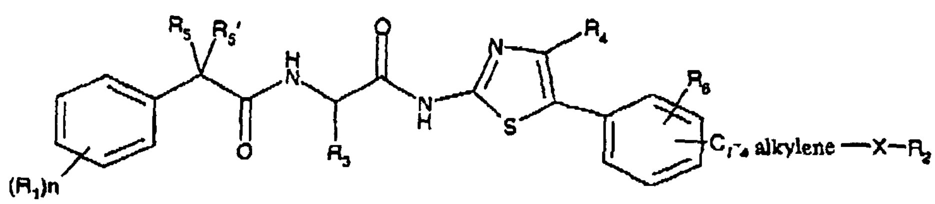

(I)

in which,
n is equal to 0, 1, 2 or 3 ;
X represents an oxygen or sulfur atom;
$R_1$ represents, independently of each other when n = 2 or 3, a halogen atom, a hydroxyl , a $C_{1-3}$ alkyl, a $C_{1-3}$ alkoxy, a trifluoromethyl, a trifluoromethyloxy or a methylenedioxy;
$R_2$ represents a $C_{1-6}$ alkyl group optionally substituted with a $C_{3-7}$ cycloalkyl group, a phenyl, a $C_{1-3}$ alkoxy group, a hydroxyl or a halogen atom; a $C_{3-7}$ cycloalkyl, piperidinyl or phenyl group;
the $C_{3-7}$ cycloalkyl, piperidinyl and phenyl groups being optionally substituted with one or more $C_{1-3}$ alkyl groups, $C_{1-3}$ alkoxy groups, a hydroxyl or a halogen atom;
$R_3$ represents a hydrogen atom or a $C_{1-6}$ alkyl group optionally substituted with a $C_{3-7}$ cycloalkyl group;
$R_4$ represents a hydrogen atom or a $C_{1-4}$ alkyl group;
$R_5$ and $R_{5'}$ represent, independently of each other, a hydrogen atom, a hydroxyl, a halogen atom, a $C_{1-3}$ alkyl

group; or $R_5$ and $R_{5'}$ form together an oxo group; and
$R_6$ represents a hydrogen atom, a halogen atom, a $C_{1-3}$ alkyl, a $C_{1-3}$ alkoxy, a trifluoromethyl or a trifluoromethoxy;
in the form of a base, of an addition salt with an acid, of a hydrate or of a solvate.

**2.** The compound as claimed in claim 1, **characterized in that**:

X represents an oxygen or sulfur atom;
$R_1$ represents, independently of each other when n = 2 or 3, a halogen atom, a methylenedioxy, a $C_{1-3}$ alkyl group, a $C_{1-3}$ alkoxy group or a trifluoromethyloxy;
$R_2$ represents a $C_{1-4}$ alkyl group optionally substituted with a $C_{4-7}$ cycloalkyl or phenyl group; a $C_{4-7}$ cycloalkyl, piperidinyl or phenyl group; the $C_{4-7}$ cycloalkyl, piperidinyl and phenyl groups being optionally substituted with one or more $C_{1-3}$ alkyl or $C_{1-3}$ alkoxy groups;
$R_3$ represents a hydrogen atom or a $C_{1-4}$ alkyl group, optionally substituted with a $C_{4-7}$ cycloalkyl group;
$R_4$ represents a hydrogen atom or a $C_{1-3}$ alkyl group;
$R_5$ and $R_{5'}$ represent, independently of each other, a hydrogen or halogen atom, a hydroxyl or a $C_{1-3}$ alkyl group; or $R_5$ and $R_{5'}$ form together an oxo group; and
$R_6$ represents a hydrogen atom or a $C_{1-3}$ alkoxy;

in the form of a base, of an addition salt with an acid, of a hydrate or of a solvate.

**3.** The compound of formula (I) as claimed in claim 1, **characterized in that**:

X represents an oxygen atom;
$R_1$ represents, independently of each other when n = 2 or 3, a halogen atom;
$R_2$ represents a $C_{1-4}$ alkyl group optionally substituted with a $C_{4-7}$ cycloalkyl or phenyl group; a $C_{4-7}$ cycloalkyl, piperidinyl or phenyl group;
the $C_{4-7}$ cycloalkyl, piperidinyl and phenyl groups being optionally substituted with 1 or 2 $C_{1-3}$ alkyl or $C_{1-3}$ alkoxy groups;
$R_3$ represents a methyl, ethyl or propyl;
$R_4$ represents a hydrogen atom or a methyl;
$R_5$ and $R_{5'}$ represent, independently of each other, a hydrogen or fluorine atom, a hydroxyl or a methyl; or $R_5$ and $R_{5'}$ form together an oxo group; and
$R_6$ represents a hydrogen atom or a methoxy;

in the form of a base, of an addition salt with an acid, of a hydrate or of a solvate.

**4.** A method for preparing a compound of formula (I) as claimed in any one of claims 1 to 3, **characterized in that**:

a peptide coupling of the compound of formula (VII)

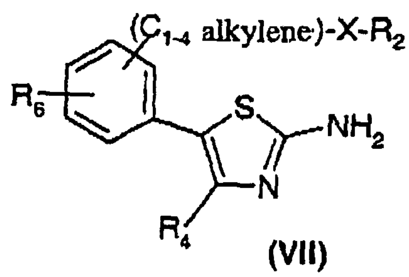

(VII)

with the amino acid of formula (VIII)

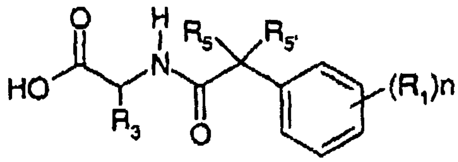

(VIII)

in which n, X, $R_1$, $R_2$, $R_3$, $R_4$, $R_5$ and $R_{5'}$ are as defined in formula (I) as claimed in claim 1, is carried out.

**5.** A method for preparing a compound of formula (I) as claimed in any one of claims 1 to 3, in which $R_3$ does not represent a hydrogen atom, by peptide coupling of an amine of formula (XIII)

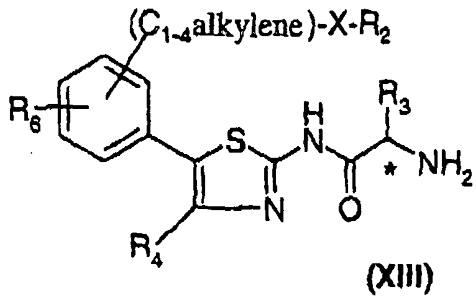

(XIII)

with an acid of formula (XIV)

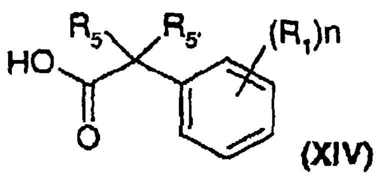

(XIV)

in which n, X, $R_1$, $R_2$, $R_3$, $R_4$, $R_5$ and $R_{5'}$ are as defined in formula (I) as claimed in claim 1.

**6.** A compound of formula (VI)

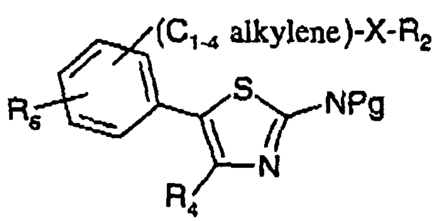

in which X, $R_2$ and $R_4$ are as defined in claim 1 and Pg represents a diphenyl ketone imine.

**7.** A compound of formula (VII)

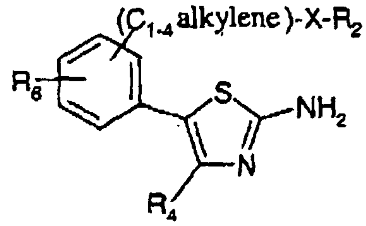 (VII)

in which X, $R_2$ and $R_4$ are as defined in claim 1.

**8.** A compound of formula (XIII)

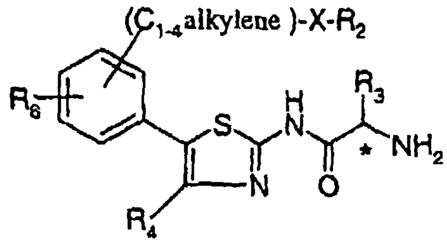 (XIII)

in which X, $R_2$, $R_3$ and $R_4$ are as defined in claim 1.

**9.** A pharmaceutical composition containing at least one compound of formula (I) as claimed in any one of claims 1 to 3, in the form of a pharmaceutically acceptable base, salt, hydrate or solvate and optionally one or more pharmaceutically acceptable excipients.

**10.** The compound of formula (I) as claimed in any one of claims 1 to 3, in the form of a pharmaceutically acceptable base, salt, hydrate or solvate, for its use as a medicament.

**11.** The use of a compound of formula (I) as claimed in any one of claims 1 to 3, in the form of a pharmaceutically acceptable base, salt, hydrate or solvate, for the preparation of a medicament for treating a pathology in which an inhibitor of the formation of β-amyloid peptide β-A4 offers a therapeutic benefit.

**12.** The use of a compound of formula (I) as claimed in any one of claims 1 to 3, in the form of a pharmaceutically acceptable base, salt, hydrate or solvate, for the preparation of a medicament for treating senile dementia, Alzheimer's disease, Down's syndrome, Parkinson's disease, amyloid angiopathy and/or cerebrovascular disorders.